# EUROPEAN PATENT APPLICATION

(11) **EP 3 978 527 A1**
(43) Date of publication of application: **06.04.2022**
(21) Application number: 20813261.3
(22) Date of filing: 27.05.2020
(51) Int. Cl.: C07K 16/28, A61P 35/00, A61P 9/12, A61K 39/00

(54) **ANTIBODY WITH ENHANCED BINDING AFFINITY FOR ENDOTHELIN RECEPTOR A**

(30) Priority: 29.05.2019 KR 20190063174; 21.05.2020 KR 20200060976
(71) Applicant: Hedgehog, Inc., Seoul, 08307 (KR)
(72) Inventor: JUNG, Sang Taek, Seongnam-si Gyeonggi-do 13562 (KR); KIM, Youn Jae, Seoul 04076 (KR); AHN, Hye-Mi, Goyang-si Gyeonggi-do 10239 (KR); KO, Byoung Joon, Cheongju-si Chungcheongbuk-do 28165 (KR); LEE, Won Kyu, Goyang-si Gyeonggi-do 10270 (KR); NA, Jung-Hyun, Wonju-si Gangwon-do 26339 (KR); JU, Man-Seok, Uijeongbu-si Gyeonggi-do 11645 (KR)
(74) Representative: V.O.
(86) International application number: PCT/KR2020/006866
(87) International publication number: WO 2020/242200

(57) **Abstract**

The present invention relates to an antibody or an antigen-binding fragment thereof that has improved binding affinity for endothelin receptor type A. The present invention also relates to an antibody or an antigen-binding fragment thereof that has improved productivity. The antibody developed in the present invention is suitable for use in the treatment and diagnosis of diseases associated with endothelin receptor type A due to its remarkably improved binding affinity for the antigen and high productivity compared to conventional antibodies.

<110>    Korea University Research and Business Foundation

<120>    Antibody with enhanced binding to endothelin receptor type A

<130>    PCT687

<150>    KR 10-2019-0063174
<151>    2019-05-29

<150>    KR 10-2020-0060976
<151>    2020-05-21

<160>    35

<170>    KoPatentIn 3.0

<210>    1
<211>    25
<212>    PRT
<213>    Artificial Sequence

<220>
<223>    VH-FR1 No.1 or 3


<400>    1



<210>    2
<211>    25
<212>    PRT
<213>    Artificial Sequence

<220>
<223>    VH-FR1 No.2


<400>    2



<210>    3
<211>    25
<212>    PRT
<213>    Artificial Sequence

<220>
<223>    VH-FR1 No.4


<400>    3

<210>    4
<211>    17
<212>    PRT
<213>    Artificial Sequence

<220>
<223>    VH-FR2 No.1

<400>    4
Ile His Trp Val Arg Gln Ala Pro Gly Lys Gly Leu Glu Trp Val Ala
1               5                   10                  15

Arg

<210>    5
<211>    17
<212>    PRT
<213>    Artificial Sequence

<220>
<223>    VH-FR2 No.2

<400>    5
Met His Trp Val Arg Gln Ala Pro Gly Lys Gly Leu Glu Trp Val Ser
1               5                   10                  15

Ala

<210>    6
<211>    17
<212>    PRT
<213>    Artificial Sequence

<220>
<223>    VH-FR2 No.3

<400>    6
Met Asn Trp Val Arg Gln Ala Pro Gly Lys Gly Leu Glu Trp Val Gly
1               5                   10                  15

Trp

<210>    7
<211>    17
<212>    PRT
<213>    Artificial Sequence

<220>
<223>    VH-FR2 No.4

<400>    7
Trp Asn Trp Ile Arg Gln Ala Pro Gly Lys Gly Leu Glu Trp Val Ala
1               5                   10                  15

Ser

<210>    8
<211>    38
<212>    PRT
<213>    Artificial Sequence

<220>
<223>    VH-FR3 No.1

<400>    8
Arg Tyr Ala Asp Ser Val Lys Gly Arg Phe Thr Ile Ser Ala Asp Thr
1               5                   10                  15

Ser Lys Asn Thr Ala Tyr Leu Gln Met Asn Ser Leu Arg Ala Glu Asp
            20                  25                  30

Thr Ala Val Tyr Tyr Cys
        35

<210>    9
<211>    38
<212>    PRT
<213>    Artificial Sequence

<220>
<223>    VH-FR3 No.2

<400>    9
Asp Tyr Ala Asp Ser Val Glu Gly Arg Phe Thr Ile Ser Arg Asp Asn
1               5                   10                  15

Ala Lys Asn Ser Leu Tyr Leu Gln Met Asn Ser Leu Arg Ala Glu Asp
            20                  25                  30

Thr Ala Val Tyr Tyr Cys
        35

<210>    10
<211>    38
<212>    PRT
<213>    Artificial Sequence

<220>
<223>    VH-FR3 No.3

<400>    10

<210>   11
<211>   38
<212>   PRT
<213>   Artificial Sequence

<220>
<223>   VH-FR3 No.4

<400>   11
Asn Tyr Ala Asp Ser Val Lys Gly Arg Phe Thr Ile Ser Arg Asp Asp
1               5                   10                  15

Ser Lys Asn Thr Phe Tyr Leu Gln Met Asn Ser Leu Arg Ala Glu Asp
            20                  25                  30

Thr Ala Val Tyr Tyr Cys
        35

<210>   12
<211>   11
<212>   PRT
<213>   Artificial Sequence

<220>
<223>   VH-FR4 No.1, 2, 3 or 4

<400>   12
Trp Gly Gln Gly Thr Leu Val Thr Val Ser Ser
1               5                   10

<210>   13
<211>   26
<212>   PRT
<213>   Artificial Sequence

<220>
<223>   VL-FR1 No.1 or 2

<400>   13

<210>   14
<211>   26
<212>   PRT
<213>   Artificial Sequence

<220>

<223>    VL-FR1 No.3

<400>    14

<210>    15
<211>    26
<212>    PRT
<213>    Artificial Sequence

<220>
<223>    VL-FR1 No.4

<400>    15

<210>    16
<211>    17
<212>    PRT
<213>    Artificial Sequence

<220>
<223>    VL-FR2 No.1

<400>    16
Val Ala Trp Tyr Gln Gln Lys Pro Gly Lys Ala Pro Lys Leu Leu Ile
1               5                   10                  15

Tyr

<210>    17
<211>    17
<212>    PRT
<213>    Artificial Sequence

<220>
<223>    VL-FR2 No.2

<400>    17
Leu Ala Trp Tyr Gln Gln Lys Pro Gly Lys Ala Pro Lys Leu Leu Ile
1               5                   10                  15

Tyr

<210>    18

<211>    17
<212>    PRT
<213>    Artificial Sequence

<220>
<223>    VL-FR2 No.3


<400>    18
Leu Asn Trp Tyr Gln Gln Lys Pro Gly Lys Ala Pro Lys Val Leu Ile
1               5                   10                  15

Tyr




<210>    19
<211>    17
<212>    PRT
<213>    Artificial Sequence

<220>
<223>    VL-FR2 No.4


<400>    19
Met Asn Trp Tyr Gln Gln Lys Pro Gly Lys Ala Pro Lys Leu Leu Ile
1               5                   10                  15

Tyr




<210>    20
<211>    36
<212>    PRT
<213>    Artificial Sequence

<220>
<223>    VL-FR3 No.1


<400>    20
Phe Leu Tyr Ser Gly Val Pro Ser Arg Phe Ser Gly Ser Arg Ser Gly
1               5                   10                  15

Thr Asp Phe Thr Leu Thr Ile Ser Ser Leu Gln Pro Glu Asp Phe Ala
            20                  25                  30

Thr Tyr Tyr Cys
        35


<210>    21
<211>    36
<212>    PRT
<213>    Artificial Sequence

<220>
<223>    VL-FR3 No.2


<400>    21

Thr Leu Gln Ser Gly Val Pro Ser Arg Phe Ser Gly Ser Gly Ser Gly
1               5                   10                  15

Thr Asp Phe Thr Leu Thr Ile Ser Ser Leu Gln Pro Glu Asp Val Ala
            20                  25                  30

Thr Tyr Tyr Cys
        35

<210>   22
<211>   36
<212>   PRT
<213>   Artificial Sequence

<220>
<223>   VL-FR3 No.3

<400>   22

<210>   23
<211>   36
<212>   PRT
<213>   Artificial Sequence

<220>
<223>   VL-FR3 No.4

<400>   23

<210>   24
<211>   11
<212>   PRT
<213>   Artificial Sequence

<220>
<223>   VL-FR4 No.1, 2, 3 or 4

<400>   24
Phe Gly Gln Gly Thr Lys Val Glu Ile Lys Arg
1               5                   10

<210>   25
<211>   8
<212>   PRT
<213>   Artificial Sequence

<220>
<223>   VH-CDR1


<400>   25
Gly Tyr Thr Phe Thr Ser Ser Asp
1               5


<210>   26
<211>   8
<212>   PRT
<213>   Artificial Sequence

<220>
<223>   VH-CDR2


<400>   26
Met Asn Pro Lys Asn Gly Asn Thr
1               5


<210>   27
<211>   6
<212>   PRT
<213>   Artificial Sequence

<220>
<223>   VL-CDR1


<400>   27
Gln Gly Ile Asn Asn Tyr
1               5


<210>   28
<211>   3
<212>   PRT
<213>   Artificial Sequence

<220>
<223>   VL-CDR2


<400>   28
Val Gly Ser
1


<210>   29
<211>   9
<212>   PRT
<213>   Artificial Sequence

<220>

<223>    VL-CDR3

<400>    29
Leu Gln His Asn Thr Tyr Pro Leu Thr
1               5

<210>    30
<211>    16
<212>    PRT
<213>    Artificial Sequence

<220>
<223>    VH-CDR3-AG8

<400>    30
Ala Lys Asp Arg Tyr Gly Ser Gly Thr Tyr Gly Phe Gly Met Asp Val
1               5                   10                  15

<210>    31
<211>    16
<212>    PRT
<213>    Artificial Sequence

<220>
<223>    VH-CDR3-EF12

<400>    31
Ala Lys Asp Pro Tyr Gly Ser Gly Thr Tyr Gly Phe Gly Met Asp Val
1               5                   10                  15

<210>    32
<211>    16
<212>    PRT
<213>    Artificial Sequence

<220>
<223>    VH-CDR3-GG12

<400>    32
Ala Lys Asp Arg Tyr Gly Ser Gly Val Tyr Gly Phe Gly Met Asp Val
1               5                   10                  15

<210>    33
<211>    16
<212>    PRT
<213>    Artificial Sequence

<220>
<223>    VH-CDR3-FG12

<400>    33

Ala Lys Asp Arg Tyr Gly Leu Gly Thr Tyr Gly Phe Gly Met Asp Val
1               5                   10                  15

<210>  34
<211>  16
<212>  PRT
<213>  Artificial Sequence

<220>
<223>  VH-CDR3-AB9

<400>  34
Ala Lys Asp Arg Tyr Gly Ser Gly Thr Tyr Gly Glu Gly Met Asp Val
1               5                   10                  15

<210>  35
<211>  227
<212>  PRT
<213>  Artificial Sequence

<220>
<223>  PFc29

<400>  35
Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Arg Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Leu
        195                 200                 205

His Glu Ala Leu His Asn His Tyr Thr Gln Lys Ser Leu Ser Leu Ser
    210                 215                 220

Pro Gly Lys
225

## Description

### Technical Field

The present invention relates to an antibody drug, and more specifically to an antibody for endothelin receptor activity modulation.

### Background Art

G-protein coupled receptors (GPCRs) are multi-transmembrane proteins with seven transmembrane domains. Upon extracellular ligand binding, G proteins dissociate from the receptors, resulting in the activation of intracellular signal transduction cascades. GPCRs play crucial roles in physiological functions such as cognition and sensation as well as responses to neurotransmitters. In addition, GPCRs not only directly regulate major biological processes (including cell growth, proliferation, migration, and death), but also are directly associated with a number of diseases such as cancer and cardiovascular diseases. For these reasons, GPCRs have attracted attention as major drug targets. GPCRs account for 30 to 50% of the currently commercially available target biomaterials for new drugs.

It is known that endothelin receptor type A (ET_{A}) is expressed mainly in the vascular smooth muscle of normal humans and causes vasoconstriction through intracellular signaling pathways when bound to its ligand, endothelin-1 (ET-1). Thus, drugs such as bosentan, an endothelin receptor antagonist, have been used as therapeutic agents for hypertension (Nature reviews drug discovery, 2011, Vol. 10, 47). Many recent basic biological and clinical studies have reported that ET_{A} is overexpressed in various cancers such as bladder cancer, lung cancer, ovarian cancer, kidney cancer, colorectal cancer, prostate cancer, breast cancer, uterine cancer, rhabdomyosarcoma, and glioblastoma and is directly involved in main cancer processes such as proliferation, migration, invasion, metastasis, and angiogenesis of cancer cells to lower the survival rate of patients. Thus, ET_{A} has been spotlighted as major anticancer targets (Nature review cancer, 2013, Vol. 13, 637).

Antibody drugs have many advantages such as high specificity, few side effects, long blood half-life, and mechanisms of action to kill defective cells (ADCC, ADCP, and CDC) by their Fc regions compared to synthetic small-molecule drugs. Nevertheless, current ET_{A}-targeted drugs are small-molecule drugs, including bosentan, zibotentan, and atrasentan, and no satisfactory antibody drugs have been reported as yet. Only a very few antibody drugs have been approved for sale even if extended to all GPCRs. Poteligeo (mogamulizumab) available from Kyowa Hakko Kirin is a CCR4-targeted antibody approved in Japan. Aimovig (erenumab) developed by Amgen in May, 2018 targets CGRP receptors and is the first U.S. FDA-approved GPCR-targeted antibody. Under these circumstances, there arises a need to develop a novel antibody that specifically binds to ET_{A} and modulates the activity of ET_{A}.

The description of the Background Art is merely provided for better understanding of the background of the invention and should not be taken as corresponding to the prior art already known to those skilled in the art.

### Detailed Description of the Invention

### Problems to be Solved by the Invention

The inventors of the present invention have earnestly and intensively conducted research to develop an antibody whose affinity and productivity are significantly improved by framework region engineering while maintaining the sequence of its antigen-binding portion capable of specific binding to previously developed endothelin receptor type A, and as a result, found an antibody that efficiently inhibits ET-1 signaling in a cell line stably expressing ET_{A}. Based on this finding, the present invention has been accomplished.

Therefore, one object of the present invention is to provide a monoclonal antibody or an antigen-binding fragment thereof including a heavy chain variable region and a light chain variable region and specifically binding to endothelin receptor type A.

A further object of the present invention is to provide a nucleic acid molecule encoding the antibody or antigen-binding fragment thereof.

Another object of the present invention is to provide a vector including the nucleic acid molecule.

Another object of the present invention is to provide a host cell including the vector.

Still another object of the present invention is to provide a composition including the antibody, the nucleic acid molecule or the vector.

Other objects and advantages of the invention become more apparent from the following detailed description, claims, and drawings.

### Means for Solving the Problems

One aspect of the present invention provides a monoclonal antibody or an antigen-binding fragment thereof including a heavy chain variable region and a light chain variable region and specifically binding to endothelin receptor type A.

The endothelin receptor type A is responsible for intracellular signaling when bound to its ligand, endothelin-1 (ET-1), and has received attention as a major target for hypertension treatment and anticancer therapy. Current ET_{A}-targeted drugs are low molecular weight compounds, including bosentan, zibotentan, and atrasentan, and there have been no reports on monoclonal antibody drugs that recognize the extracellular domain of endothelin receptor type A as an antigen.

The term "epitope" as used herein refers to a localized region of an antigen to which an antibody or an antigen-binding fragment thereof can specifically bind. An epitope can be, for example, contiguous amino acids of a polypeptide as an antigen or an epitope can, for example, come together from two or more non-contiguous regions of a polypeptide or polypeptides by tertiary folding. An epitope can include at least 2, 3, 4, 5, 6, 7, 8, 9, 10, 11, 12, 13, 14, or 15 contiguous or non-contiguous amino acids in a unique spatial conformation. The antibody or fragment thereof of the present invention recognizes and specifically binds to the extracellular domain of a GPCR (e.g., endothelin receptor type A) as an antigen wherein the extracellular domain of the GPCR includes one N-term and three ECLs (ECL1, ECL2, and ECL3). That is, the antibody or fragment thereof of the present invention can bind specifically to at least one extracellular domain selected from the group consisting of N-term, ECL1, ECL2, and ECL3 as an epitope. The epitope may include one or more amino acids.

Methods for determining what epitopes are bound by a given antibody (*i.e*., epitope mapping) are known in the art and include, for example, immunoblotting and immunoprecipitation assays through testing for reactivity with an antibody. Methods of determining spatial conformation of epitopes include techniques in the art and those described herein, for example, x-ray crystallography, 2-dimensional nuclear magnetic resonance and HDX-MS (*see*, *e.g*., Epitope Mapping Protocols in Methods in Molecular Biology, Vol. 66, G. E. Morris, Ed. (1996)).

According to a preferred embodiment of the present invention, the epitope to which the antibody or antigen-binding fragment thereof of the present invention binds can be determined by, *e.g*., NMR spectroscopy, X-ray diffraction crystallography studies, ELISA assays, hydrogen/deuterium exchange coupled with mass spectrometry (HDX-MS), array-based oligo-peptide scanning assays, and/or mutagenesis mapping (e.g., Giege R et al., (1994) Acta Crystallogr D Biol Crystallogr 50(Pt 4): 339-350; McPherson A (1990) Eur J Biochem 189: 1-23; Chayen E (1997) Structure 5: 1269-1274; McPherson A (1976) J Biol Chem 251:6300-6303).

Antibodies can be of any type (*e.g*., IgG, IgE, IgM, IgD, IgA, or IgY) or any subclass (e.g., IgG1, IgG2, IgG3, and IgG4 in humans; and IgG1, IgG2a, IgG2b, and IgG3 in mice) of immunoglobulin molecule. Immunoglobulins, *e*.*g*., IgG1, exist in several allotypes. The term "antibody" as used herein is intended to include commonly known isotypes and allotypes. The antibodies described herein are of the IgG1, IgG2, IgG3, or IgG4 subclass or any hybrid thereof (*e.g*., a hybrid of IgG2 and IgG4).

The term "monoclonal antibody" as used herein refers to an antibody that displays a single binding specificity and affinity for a particular epitope.

The monoclonal antibody is herein meant to include its fragments. Preferably, the fragments mean antigen binding fragments. The fragment can be prepared by various methods known in the art. For example, Fab and F(ab')2 fragments can be produced by proteolytic cleavage of immunoglobulin molecules, using enzymes such as papain (to produce Fab fragments) or pepsin (to produce F(ab')2 fragments).

The term "fragment" may be a Fab, a Fab', a F(ab')2, a Fv, a single chain Fv (scFv), or a sdAb consisting of a monomeric heavy chain variable chain (VH) or light chain variable region (VL) domain. Such fragments are well known in the art.

A VH domain, or one or more complementarity-determining regions (CDRs) thereof, described herein can be linked to a constant domain for forming a heavy chain. Similarly, a VL domain, or one or more CDRs thereof, described herein can be linked to a constant domain for forming a light chain. A full length heavy chain and full length light chain combine to form a full length antibody.

The term "recombinant human antibody," as used herein, includes all human antibodies that are prepared, expressed, created or isolated by recombinant means, such as (a) antibodies isolated from an animal (*e.g*., a mouse) that is transgenic or transchromosomal for human immunoglobulin genes or a hybridoma prepared therefrom, (b) antibodies isolated from a host cell transformed to express the antibody, *e*.*g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies comprise variable and constant regions that utilize particular human germline immunoglobulin sequences encoded by the germline genes, but include subsequent rearrangements and mutations which occur, for example, during antibody maturation. As known in the art (*see*, *e.g*., Lonberg (2005) Nature Biotech. 23(9): 1117-1125), the variable region contains the antigen binding domain, which is encoded by various genes that rearrange to form an antibody specific for a foreign antigen. In addition to rearrangement, the variable region can be further modified by multiple single amino acid changes to increase the affinity of the antibody to the foreign antigen. Therefore, the rearranged and somatically mutated nucleic acid molecules that encode the light chain and heavy chain immunoglobulin polypeptides in response to an antigen cannot have sequence identity with the original nucleic acid molecules, but instead will be substantially identical or similar (*i.e*., have at least 80% identity).

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The antibodies described herein can include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences. The terms "human" antibodies and "fully human" antibodies are used synonymously.

A "humanized" antibody refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

According to a preferred embodiment of the present invention, the heavy chain variable region of the antibody or antigen-binding fragment thereof includes three complementarity-determining regions (CDRs): VH-CDR1, VH-CDR2, and VH-CDR3.

According to a preferred embodiment of the present invention, the heavy chain variable region of the antibody or antigen-binding fragment thereof includes VH-CDR1 including the sequence set forth in SEQ ID NO: 25 and VH-CDR2 including the sequence set forth in SEQ ID NO: 26.

According to a preferred embodiment of the present invention, the heavy chain variable region of the antibody or antigen-binding fragment thereof includes VH-CDR3 including the sequence set forth in SEQ ID NO: 30 or VH-CDR3 including a sequence including mutations at one or more positions selected from the group consisting of amino acid positions 4, 7, 9, and 12 in the sequence of SEQ ID NO: 30.

According to a preferred embodiment of the present invention, the VH-CDR3 of the heavy chain variable region of the antibody or antigen-binding fragment thereof includes one or more mutations wherein the mutations include a substitution of the amino acid at position 4 in the sequence of SEQ ID NO: 30 to proline (P), a substitution of the amino acid at position 7 in the sequence of SEQ ID NO: 30 to leucine (L), a substitution of the amino acid at position 9 in the sequence of SEQ ID NO: 30 to valine (V) or a substitution of the amino acid at position 12 in the sequence of SEQ ID NO: 30 to glutamate (E).

According to a preferred embodiment of the present invention, the VH-CDR3 of the heavy chain variable region of the antibody or antigen-binding fragment thereof includes an amino acid sequence selected from the group consisting of those set forth in SEQ ID NOs: 30, 31, 32, 33, and 34.

According to a preferred embodiment of the present invention, the light chain variable region of the antibody or antigen-binding fragment thereof includes three complementarity-determining regions (CDRs): VL-CDR1, VL-CDR2, and VL-CDR3.

According to a preferred embodiment of the present invention, the light chain variable region of the antibody or antigen-binding fragment thereof includes VL-CDR1 including the sequence set forth in SEQ ID NO: 27, VL-CDR2 including the sequence set forth in SEQ ID NO: 28, and VL-CDR3 including the sequence set forth in SEQ ID NO: 29.

According to a preferred embodiment of the present invention, the heavy chain variable region of the antibody or antigen-binding fragment thereof includes four framework regions: VH-FR1, VH-FR2, VH-FR3, and VH-FR4.

According to a preferred embodiment of the present invention, the framework regions and the CDRs of the heavy chain variable region of the antibody or antigen-binding fragment thereof have the following arrangement: VH-FR1/VH-CDR1/VH-FR2/VH-CDR2/VH-FR3/VH-CDR3/VH-FR4.

According to a preferred embodiment of the present invention, the heavy chain variable region of the antibody or antigen-binding fragment thereof includes a framework region 1 (VH-FR1) including the sequence set forth in SEQ ID NO: 1 or a sequence including mutations at one or more positions selected from the group consisting of amino acid positions 16 and 24 in the sequence of SEQ ID NO: 1.

According to a preferred embodiment of the present invention, the VH-FR1 of the heavy chain variable region of the antibody or antigen-binding fragment thereof includes one or more mutations wherein the mutations include a substitution of the amino acid at position 16 in the sequence of SEQ ID NO: 1 to arginine (R) or a substitution of the amino acid at position 24 in the sequence of SEQ ID NO: 1 to valine (V).

According to a preferred embodiment of the present invention, the VH-FR1 of the heavy chain variable region of the antibody or antigen-binding fragment thereof includes an amino acid sequence selected from the group consisting of those set forth in SEQ ID NOs: 1, 2, and 3.

According to a preferred embodiment of the present invention, the heavy chain variable region of the antibody or antigen-binding fragment thereof includes a framework region 2 (VH-FR2) including the sequence set forth in SEQ ID NO: 4 or a sequence including mutations at one or more positions selected from the group consisting of amino acid positions 1, 2, 4, 16, and 17 in the sequence of SEQ ID NO: 4.

According to a preferred embodiment of the present invention, the VH-FR2 of the heavy chain variable region of the antibody or antigen-binding fragment thereof includes one or more mutations selected from the group consisting of mutations including a substitution of the amino acid at position 1 in the sequence of SEQ ID NO: 4 to methionine (M), mutations including a substitution of the amino acid at position 2 in the sequence of SEQ ID NO: 4 to asparagine (N), mutations including a substitution of the amino acid at position 4 in the sequence of SEQ ID NO: 4 to isoleucine (I), mutations including a substitution of the amino acid at position 16 in the sequence of SEQ ID NO: 4 to serine (S) or glycine (G), and mutations including a substitution of the amino acid at position 17 in the sequence of SEQ ID NO: 4 to alanine (A), tryptophan (W) or serine (S).

According to a preferred embodiment of the present invention, the VH-FR2 of the heavy chain variable region of the antibody or antigen-binding fragment thereof includes an amino acid sequence selected from the group consisting of those set forth in SEQ ID NOs: 4, 5, 6, and 7.

According to a preferred embodiment of the present invention, the heavy chain variable region of the antibody or antigen-binding fragment thereof includes a framework region 3 (VH-FR3) including the sequence set forth in SEQ ID NO: 8 or a sequence including mutations at one or more positions selected from the group consisting of amino acid positions 1, 4, 5, 6, 7, 8, 12, 14, 16, 17, 19, 20, and 21 in the sequence of SEQ ID NO:8.

According to a preferred embodiment of the present invention, the VH-FR3 of the heavy chain variable region of the antibody or antigen-binding fragment thereof includes one or more mutations selected from the group consisting of mutations including a substitution of the amino acid at position 1 in the sequence of SEQ ID NO: 8 to aspartate (D), threonine (T) or asparagine (N), mutations including a substitution of the amino acid at position 4 in the sequence of SEQ ID NO: 8 to alanine (A), mutations including a substitution of the amino acid at position 5 in the sequence of SEQ ID NO: 8 to aspartate (D), mutations including a substitution of the amino acid at position 6 in the sequence of SEQ ID NO: 8 to phenylalanine (F), mutations including a substitution of the amino acid at position 7 in the sequence of SEQ ID NO: 8 to glutamate (E), mutations including a substitution of the amino acid at position 8 in the sequence of SEQ ID NO: 8 to arginine (R), mutations including a substitution of the amino acid at position 12 in the sequence of SEQ ID NO: 8 to phenylalanine (F), mutations including a substitution of the amino acid at position 14 in the sequence of SEQ ID NO: 8 to arginine (R) or leucine (L), mutations including a substitution of the amino acid at position 16 in the sequence of SEQ ID NO: 8 to asparagine (N) or aspartate (D), mutations including a substitution of the amino acid at position 17 in the sequence of SEQ ID NO: 8 to alanine (A), mutations including a substitution of the amino acid at position 19 in the sequence of SEQ ID NO: 8 to serine (S), mutations including a substitution of the amino acid at position 20 in the sequence of SEQ ID NO: 8 to serine (S), and mutations including a substitution of the amino acid at position 21 in the sequence of SEQ ID NO: 8 to leucine (L) or phenylalanine (F).

According to a preferred embodiment of the present invention, the VH-FR3 of the heavy chain variable region of the antibody or antigen-binding fragment thereof includes an amino acid sequence selected from the group consisting of those set forth in SEQ ID NOs: 8, 9, 10, and 11.

According to a preferred embodiment of the present invention, the heavy chain variable region of the antibody or antigen-binding fragment thereof includes a framework region 4 (VH-FR4) including the sequence set forth in SEQ ID NO: 12.

According to a preferred embodiment of the present invention, the light chain variable region of the antibody or antigen-binding fragment thereof includes four framework regions: VL-FR1, VL-FR2, VL-FR3, and VL-FR4.

According to a preferred embodiment of the present invention, the framework regions and the CDRs of the light chain variable region of the antibody or antigen-binding fragment thereof have the following arrangement: VL-FR1/VL-CDR1/VL-FR2/VL-CDR2/VL-FR3/VL-CDR3/VL-FR4.

According to a preferred embodiment of the present invention, the light chain variable region of the antibody or antigen-binding fragment thereof includes a framework region 1 (VL-FR1) including the sequence set forth in SEQ ID NO: 13 or a sequence including mutations at one or more positions selected from the group consisting of amino acid positions 4 and 24 in the sequence of SEQ ID NO: 13.

According to a preferred embodiment of the present invention, the VL-FR1 of the light chain variable region of the antibody or antigen-binding fragment thereof includes one or more mutations wherein the mutations include a substitution of the amino acid at position 4 in the sequence of SEQ ID NO: 13 to leucine (L) or a substitution of the amino acid at position 24 in the sequence of SEQ ID NO: 13 to serine (S).

According to a preferred embodiment of the present invention, the VL-FR1 of the light chain variable region of the antibody or antigen-binding fragment thereof includes an amino acid sequence selected from the group consisting of those set forth in SEQ ID NOs: 13, 14, and 15.

According to a preferred embodiment of the present invention, the light chain variable region of the antibody or antigen-binding fragment thereof includes a framework region 2 (VL-FR2) including the sequence set forth in SEQ ID NO: 16 or a sequence including mutations at one or more positions selected from the group consisting of amino acid positions 1, 2, and 14 in the sequence of SEQ ID NO: 16.

According to a preferred embodiment of the present invention, the VL-FR2 of the light chain variable region of the antibody or antigen-binding fragment thereof includes one or more mutations selected from the group consisting of mutations including a substitution of the amino acid at position 1 in the sequence of SEQ ID NO: 16 to leucine (L) or methionine (M), mutations including a substitution of the amino acid at position 2 in the sequence of SEQ ID NO: 16 to asparagine (N), and mutations including a substitution of the amino acid at position 14 in the sequence of SEQ ID NO: 16 to valine (V).

According to a preferred embodiment of the present invention, the VL-FR2 of the light chain variable region of the antibody or antigen-binding fragment thereof includes an amino acid sequence selected from the group consisting of those set forth in SEQ ID NOs: 16, 17, 18, and 19.

According to a preferred embodiment of the present invention, the light chain variable region of the antibody or antigen-binding fragment thereof includes a framework region 3 (VL-FR3) including the sequence set forth in ID NO: 20 or a sequence including mutations at one or more positions selected from the group consisting of amino acid positions 1, 3, 14, and 31 in the sequence of SEQ ID NO: 20.

According to a preferred embodiment of the present invention, the VL-FR3 of the light chain variable region of the antibody or antigen-binding fragment thereof includes one or more mutations selected from the group consisting of mutations including a substitution of the amino acid at position 1 in the sequence of SEQ ID NO: 20 to threonine (T), serine (S) or tyrosine (Y), mutations including a substitution of the amino acid at position 3 in the sequence of SEQ ID NO: 20 to glutamine (Q), histidine (H) or glutamate (E), mutations including a substitution of the amino acid at position 14 in the sequence of SEQ ID NO: 20 to glycine (G), and mutations including a substitution of the amino acid at position 31 in the sequence of SEQ ID NO: 20 to valine (V).

According to a preferred embodiment of the present invention, the VL-FR3 of the light chain variable region of the antibody or antigen-binding fragment thereof includes an amino acid sequence selected from the group consisting of those set forth in SEQ ID NOs: 20, 21, 22, and 23.

According to a preferred embodiment of the present invention, the light chain variable region of the antibody or antigen-binding fragment thereof includes a framework region 4 (VL-FR4) including the sequence set forth in SEQ ID NO: 24.

A further aspect of the present invention provides a nucleic acid molecule encoding the antibody or antigen-binding fragment thereof, a vector including the nucleic acid molecule or a host cell including the vector.

The nucleic acid molecule of the present invention may be an isolated or recombinant one. Examples of such nucleic acid molecules include single- and doublestranded DNA and RNA and their corresponding complementary sequences. The isolated nucleic acid may be isolated from a naturally occurring source. In this case, the isolated nucleic acid is separated from the peripheral gene sequence present in the genome of a subject from which the nucleic acid is to be isolated. The isolated nucleic acid may be a nucleic acid, for example, a PCR product, a cDNA molecule or an oligonucleotide, that is enzymatically or chemically synthesized from a template. In this case, the nucleic acid produced from this procedure can be understood as the isolated nucleic acid molecule. The isolated nucleic acid molecule represents a nucleic acid molecule in the form of a separate fragment or as a component of a larger nucleic acid construct. A nucleic acid is "operably linked" when arranged in a functional relationship with another nucleic acid sequence. For example, the DNA of a presequence or secretory leader is operably linked to the DNA of the polypeptide when expressed as a preprotein, which is a presecretory polypeptide. A promoter or an enhancer affecting the transcription of the polypeptide sequence is operably linked to a coding sequence or a ribosome-binding site is operably linked to a coding sequence when it is arranged such that translation is promoted. Generally, the term "operably linked" means that DNA sequences to be linked are located adjacent to each other. In the case of secretory leaders, the term "operably linked" means that the secretory leaders are present adjacent to each other in the same leading frame. However, an enhancer needs not be contiguous. The linkage is performed by ligation at a convenient restriction enzyme site. In the case where this site does not exist, a synthetic oligonucleotide adaptor or a linker is used according to a suitable method known in the art.

As used herein, the term "vector" is used to refer to a carrier into which a nucleic acid sequence can be inserted for introduction into a cell where it can be replicated. A nucleic acid sequence may be "exogenous," or "heterologous". Examples of such vectors include, but are not limited to, plasmids, cosmids, and viruses (e.g., bacteriophage). One of skill in the art may construct a vector through standard recombinant techniques (Maniatis et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

As used herein, the term "expression vector" refers to a vector containing a nucleic acid sequence coding for at least part of a gene product capable of being transcribed. In some cases, RNA molecules are then translated into a protein, polypeptide, or peptide. Expression vectors can contain a variety of regulatory sequences. In addition to regulatory sequences that govern transcription and translation, vectors and expression vectors may contain nucleic acid sequences that serve other functions as well.

As used herein, the term "host cell" refers to any transgenic organism that is capable of replicating the vector or expressing the gene encoded by the vector. Suitable organisms include eukaryotes and prokaryotes. The host cell may be transfected or transformed by the vector. The transfection or transformation refers to a process for transferring or introducing the exogenous nucleic acid molecule into the host cell.

The host cell of the present invention is preferably a bacterial cell, CHO cell, HeLa cell, HEK293 cell, BHK-21 cell, COS7 cell, COP5 cell, A549 cell or NIH3T3 cell, but is not limited thereto.

Another aspect of the present invention provides a composition including the antibody or fragment thereof, the nucleic acid molecule or the vector.

According to a preferred embodiment of the present invention, the composition is a pharmaceutical composition for preventing or treating hypertension or cancer.

The pharmaceutical composition of the present invention may include (a) the antibody or antigen-binding fragment thereof, the nucleic acid molecule or the vector including the nucleic acid molecule and (b) a pharmaceutically acceptable carrier.

Another aspect of the present invention provides a method for preventing or treating hypertension or cancer including administering the pharmaceutical composition to a subj ect.

The type of the cancer to be prevented or treated by the pharmaceutical composition of the present invention is not limited. The pharmaceutical composition of the present invention can be administered to treat a variety of cancers, including: leukemias; lymphomas such as acute lymphocytic leukemia, acute nonlymphocytic leukemias, chronic lymphocytic leukemia, chronic myelogenous leukemia, Hodgkin's disease, non-Hodgkin's lymphomas, and multiple myeloma; childhood solid tumors such as brain tumors, glioblastoma, neuroblastoma, rhabdomyosarcoma, retinoblastoma, Wilms tumor, bone tumors, and soft-tissue sarcomas; and common solid tumors of adults such as lung cancer, breast cancer, prostate cancer, urinary cancers, uterine cancers, oral cancers, pancreatic cancer, melanoma and other skin cancers, stomach cancer, colon cancer, ovarian cancer, brain tumors, liver cancer, laryngeal cancer, thyroid cancer, esophageal cancer, and testicular cancer.

The pharmaceutically acceptable carriers are those that are commonly used for formulation. Examples of the pharmaceutically acceptable carriers include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present invention may further include one or more additives selected from the group consisting of lubricating agents, wetting agents, sweetening agents, flavoring agents, emulsifying agents, suspending agents, and preservatives. Details of suitable pharmaceutically acceptable carriers and formulations can be found in Remington's Pharmaceutical Sciences (19th ed., 1995).

The pharmaceutical composition of the present invention can be administered orally or parenterally, preferably parenterally. Examples of suitable parenteral routes of administration include intravenous injection, local injection, and intraperitoneal injection.

A suitable dose of the pharmaceutical composition according to the present invention may vary depending on factors such as formulation, mode of administration, patient's age, weight, sex, pathological condition, and diet, time of administration, route of administration, excretion rate, and responsiveness. A skilled physician can easily determine and prescribe a dose of the pharmaceutical composition according to the present invention effective for desired treatment or prevention. According to a preferred embodiment of the present invention, the pharmaceutical composition is administered in a daily dose of 0.0001 to 100 mg/kg.

The pharmaceutical composition of the present invention can be formulated with one or more pharmaceutically acceptable carriers and/or excipients in accordance with methods that can be easily carried out by those skilled in the art. The pharmaceutical composition can be provided in unit dosage forms or dispensed in multi-dose containers. The formulation may be in the form of a solution, suspension or emulsion in an oil or aqueous medium or may be in the form of an extract, powder, granule, tablet or capsule. The formulation may further include a dispersant or a stabilizer.

The pharmaceutical composition of the present invention may be used alone or in combination with one or more other conventional chemotherapies or radiotherapies. This combination therapy is more effective in treating cancer. One or more chemotherapeutic agents can be used in combination with the composition of the present invention. Examples of the chemotherapeutic agents include cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, and methotrexate. The radiotherapies used in combination with the composition of the present invention may be, for example, X-ray irradiation and γ-ray irradiation.

Another aspect of the present invention provides a method for quantifying endothelin receptor type A in a sample, including treating the sample with the antibody or antigen-binding fragment thereof.

The antibody or antigen-binding fragment thereof of the present invention can be used to accurately measure the amount of endothelin receptor type A in a sample due to its ability to specifically bind to the endothelin receptor type A.

Another aspect of the present invention provides a method for providing information necessary for the diagnosis of a disease caused by overexpression of endothelin receptor type A, including (a) separating a sample from a subject, (b) treating the sample with the antibody or antigen-binding fragment thereof, and (c) determining whether the expression level of endothelin receptor type A in the sample from the subject is higher than that of endothelin receptor type A in a normal sample.

Endothelin receptor type A binds to its ligand ET-1 to cause vasoconstriction (Nature reviews drug discovery, 2011, Vol. 10, 47). In addition, endothelin receptor type A is overexpressed in a variety of cancers such as bladder cancer, lung cancer, ovarian cancer, kidney cancer, colorectal cancer, prostate cancer, breast cancer, uterine cancer, rhabdomyosarcoma, and glioblastoma and is directly involved in main cancer processes such as proliferation, migration, invasion, metastasis, and angiogenesis of cancer cells (Nature review cancer, 2013, Vol. 13, 637). For these reasons, a comparison of the expression level of endothelin receptor type A in the sample with that in a sample from a normal human can provide information necessary for the diagnosis of a disease caused by overexpression of endothelin receptor type A.

According to a preferred embodiment of the present invention, the disease caused by overexpression of endothelin receptor type A is cancer or hypertension.

Yet another aspect of the present invention provides a kit for quantifying endothelin receptor type A including the antibody or antigen-binding fragment thereof.

The quantification kit of the present invention can quantify the amount of endothelin receptor type A by analyzing an antigen to the antibody through an antigen-antibody binding reaction. The antigen-antibody binding reaction is preferably selected from the group consisting of, but not limited to, enzyme-linked immunosorbent assay (ELISA), radioimmunoassay (RIA), sandwich assay, Western blotting on polyacrylamide gel, immunoblotting assay, and immunohistochemical staining.

A support for the antigen-antibody binding reaction is selected from the group consisting of, but not limited to, nitrocellulose membranes, PVDF membranes, well plates made of polyvinyl or polystyrene resin, and slide glasses.

The secondary antibody is preferably labeled with a color developing reagent known in the art. The color developing reagent can be selected from the group consisting of fluoresceins and dyes. The fluoresceins may be, for example, horseradish peroxidase (HRP), alkaline phosphatase, colloid gold, poly-L-lysine-fluorescein isothiocyanate (FITC), and rhodamine-B-isothiocyanate (RITC). A substrate for inducing color development is preferably used depending on the color developing reagent. The substrate is preferably selected from the group consisting of, but not limited to, 3,3',5,5'-tetramethylbenzidine (TMB), 2,2'-azino-bis(3-ethylbenzothiazoline)-6-sulfonic acid (ABTS), and ophenylenediamine (OPD).

### Effects of the Invention

The features and advantages of the present invention are summarized as follows.
(i) The antibody or antigen-binding fragment thereof of the present invention has improved binding affinity for endothelin receptor type A.
(ii) The antibody or antigen-binding fragment thereof of the present invention binds to endothelin receptor type A with improved productivity.
(iii) The antibody developed in the present invention is suitable for use in the treatment and diagnosis of diseases associated with endothelin receptor type A due to its remarkably improved binding affinity for the antigen and high productivity compared to conventional antibodies.

### Brief Description of the Drawings

Fig. 1 shows constructed expression vectors for animal cells.
Fig. 2 shows the amino acid sequences of four antibodies with engineered framework regions.
Fig. 3 shows the results of analysis of four antibodies with engineered framework regions after purification in animal cells.
Fig. 4 shows the yield of MJ-F1, which selectively binds to endothelin receptor type A and whose framework regions were engineered to have trastuzumab framework sequences, in animal cells.
Fig. 5 shows the results of ELISA for four antibodies with engineered framework regions to human endothelin receptor type A.
Fig. 6 shows the production of five antibodies, which selectively bind to endothelin receptor type A and whose framework regions were engineered to have trastuzumab framework sequences, in animal cells.
Fig. 7 shows the results of ELISA for five antibodies, which have trastuzumab framework sequences and selectively bind to human endothelin receptor type A as an antigen.
Figs. 8A and 8B show the results of ELISA for five antibodies, which have trastuzumab framework sequences and selectively bind to human endothelin receptor type A, and pH-dependent human FcRn pH 7.4 (Fig. 8A), pH 6.0 (Fig. 8B).
Figs. 9A to 9D show the results of ELISA for five antibodies, which have trastuzumab framework sequences and selectively bind to human endothelin receptor type A, and human FcyRIIa variants FcγRIIa-131R (Fig. 9A), FcγRIIa-131H (Fig. 9B), FcγRIIa-158V (Fig. 9C), FcγRIIa-158F (Fig. 9D).
Fig. 10 shows the results of ELISA for five antibodies, which have trastuzumab framework sequences and selectively bind to human endothelin receptor type A, and human C1q.
Figs. 11A and 11B show the inhibitory efficacies of four antibodies, all of which have trastuzumab framework sequences and selectively bind to human endothelin receptor type A, on the proliferation of colorectal cancer cell lines HT-29 (Fig. 11A) and HCT-116 (Fig. 11B).
Fig. 12A shows anticancer activities of four antibodies, all of which have trastuzumab framework sequences and selectively bind to human endothelin receptor type A, after administration to colorectal cancer xenograft models and Fig. 12B shows colorectal cancer tissues excised from the colorectal cancer xenograft models administered the antibodies.
Figs. 13A and 13B show the inhibitory efficacies of four antibodies, all of which have trastuzumab framework sequences and selectively bind to human endothelin receptor type A, on the proliferation of pancreatic cancer cell lines AsPC-1 (Fig. 13A) and Panc-1 (Fig. 13B).
Figs. 14A and 14B show the inhibitory efficacies of four antibodies, all of which have trastuzumab framework sequences and selectively bind to human endothelin receptor type A, on the proliferation of gastric cancer cell lines SNU-216 (Fig. 14A) and SNU-668 (Fig. 14B).
Fig. 15 shows the results of SEC-HPLC for four antibodies, all of which have trastuzumab framework sequences and selectively bind to human endothelin receptor type A.
Fig. 16 shows the results of glycan profiling for four antibodies, all of which have trastuzumab framework sequences and selectively bind to human endothelin receptor type A.
Fig. 17 shows the results of RP-HPLC for four antibodies, all of which have trastuzumab framework sequences and selectively binding to human endothelin receptor type A.
Figs. 18A, 18B, 18C, and 18D show the thermal stability test results for MJ-F1-WT, MJ-F1-pFc29, GG12-pFc29, and FG12-pFc29, all of which have trastuzumab framework sequences and selectively bind to human endothelin receptor type A, respectively.
Fig. 19 shows the inhibitory activities of the antibodies (each 200 nM) described in Korean Patent Application No. 10-2017-0075129 on ET-1 (10 nM)-induced signaling in cells of the colorectal cancer cell line HT-29 overexpressing ET_{A}.

### Mode for Carrying out the Invention

The present invention will be more specifically explained with reference to the following examples. It will be evident to those skilled in the art that the scope of the present invention is not limited by these examples according to the gist of the present invention.

### EXAMPLES

### <Example 1> Production of antibodies with engineered framework regions based on sequences of complementarity-determining regions (CDRs) of antibodies specifically binding to endothelin receptor type A

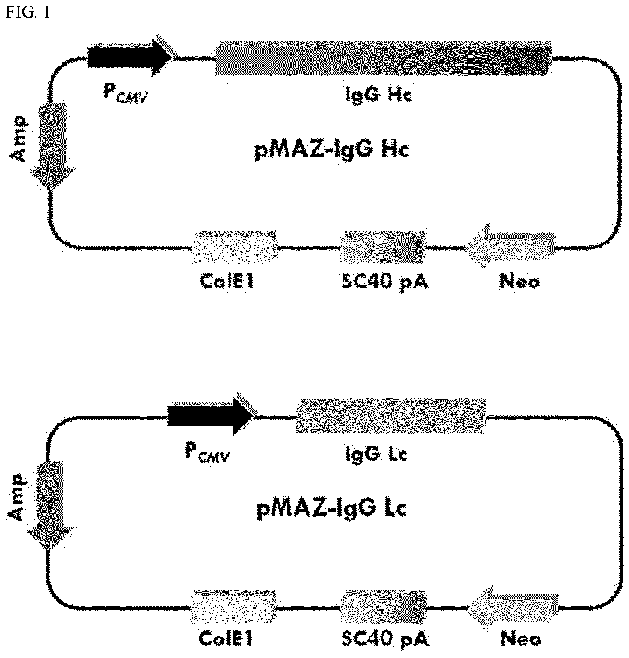

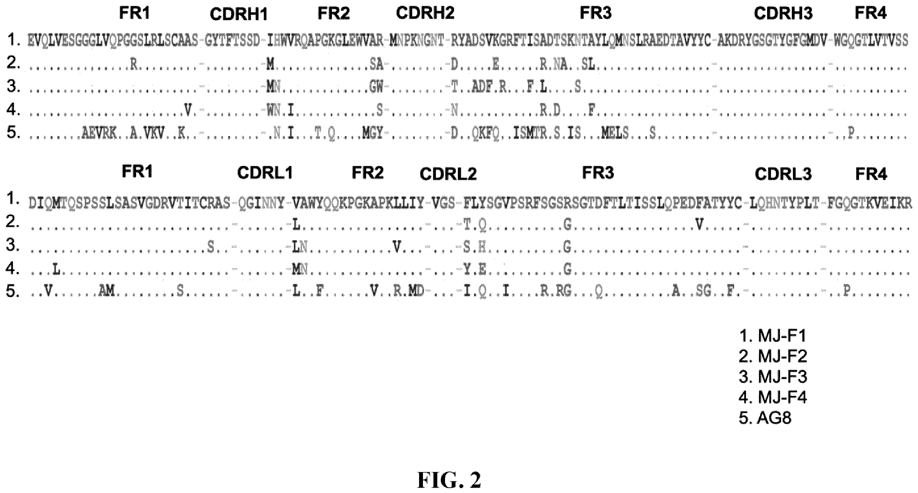

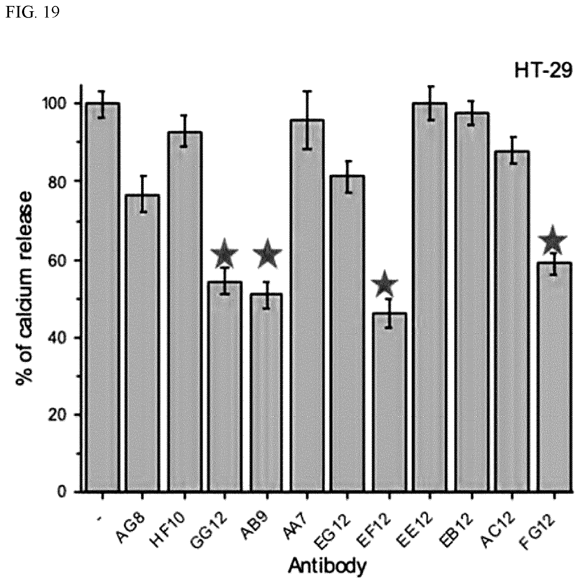

In this example, antibodies with improved stability and productivity were produced using optimal combinations of the framework region sequences of various antibody drugs and the CDR sequences of the ET_{A}-specific antibodies (AG8, EF12, GG12, FG12, AB9) described in Korean Patent Application No. 10-2017-0075129 (Fig. 19) based on the sequences of the ET_{A}-specific antibodies. To this end, the framework regions of four therapeutic antibodies (trastuzumab, bevacizumab, omalizumab, and adalimumab) were overlapped with the complementarity-determining regions (CDRs) of the heavy and light chain variable regions of the ET_{A}-specific antibodies, which are known to play an important role in antigen-antibody interactions, by PCR. The four amplified heavy chain variable region genes and the four amplified light chain variable region genes were digested with the restriction enzymes XbaI/BssHII and inserted into expression vectors (pMAZ) for animal cells, which had been digested with the same restriction enzymes (Fig. 1). The fused antibody expression vectors were transformed into *Escherichia coli* Jude1 (*F- mcrA Δ(mrr-hsdRMS-mcrBC)* ϕ*80lacZΔM15 ΔlacX74 recA1 endA1 araD139 Δ(ara, leu)7697 galU galK λ- rpsL nupG)* and the DNA sequences of the constructed expression vectors were validated by Sanger sequencing. The sequences of the four produced antibodies (MJ-F1, MJ-F2, MJ-F3, and MJ-F4) are shown in Fig. 2.

### <Example 2> Expression and purification of the four antibodies with engineered framework regions in animal cells

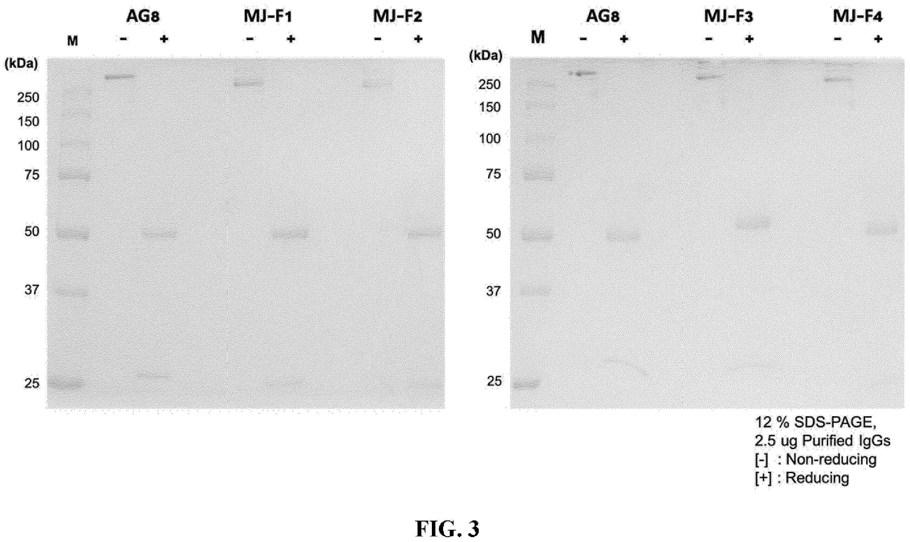

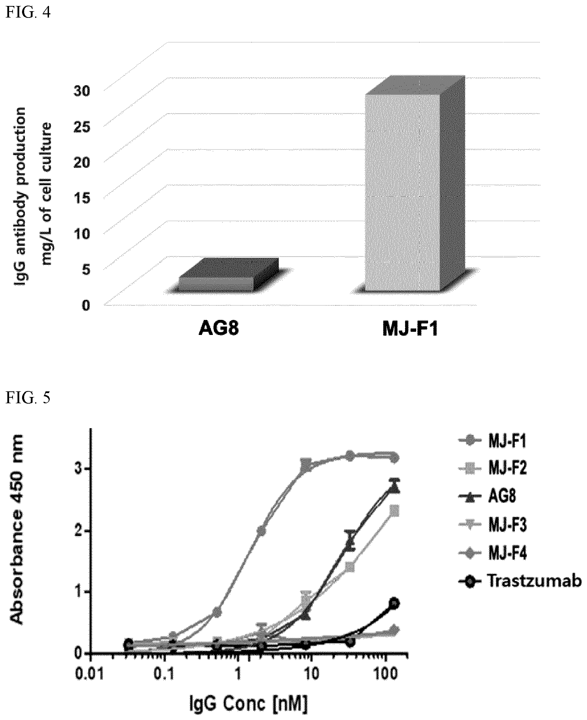

After transformation into *E. coli,* the amplified expression vectors were recovered and transiently transfected into Expi293 cells using a 150 mM NaCl solution containing polyethyleneimine (PEI, Polysciences, USA). Cells were cultured in Freestyle293 expression medium (Life Technologies, USA) at a temperature of 37 °C for 7 days. The expressed cell culture was centrifuged at 6,000 rpm for 20 min. The supernatant was collected and passed through a 0.22 µm filter. The filtrate was allowed to bind to 1 ml of Protein A resin (Amicogen, Korea) at 4 °C for 16 h. The bound resin was washed with 10 CV (column volume) of PBS solution, eluted with 100 mM glycine solution (pH 2.7), and neutralized with 1 M Tris-HCl (pH 8.0). After buffer change with PBS, the sizes and purities of the light and heavy chains of the four purified antibodies were analyzed by SDS-PAGE under reducing and non-reducing conditions (Fig. 3). The yields of AG8 and the four purified antibodies (MJ-F1, MJ-F2, MJ-F3, and MJ-F4) were compared and analyzed. The yield of MJ-F1 was 15 times higher than that of AG8 (Fig. 4).

### <Example 3> Analysis of binding affinities between extracellularly exposed regions of endothelin receptor type A as antigen and the produced antibodies

In this example, the binding affinities of AG8 and the four prepared antibodies (MJ-F1, MJ-F2, MJ-F3, and MJ-F4) for endothelin receptor type A were analyzed by ELISA. To this end, human G_{αi3} was diluted with 0.05 M Na₂CO₃ (pH 9.6) as a buffer solution and allowed to bind to 96-well plates (Costar) at a concentration of 5 µg/well at 4 °C for 16 h. Each of the 96-well plates was added to a PBST solution (a PBS solution containing 0.05% Tween-20) containing 4% skim milk such that the concentration was 5% (w/v), followed by blocking at room temperature for 1 h. After the solution was discarded, the 96-well plate was washed 4 times with a PBS solution containing 0.05% Tween-20 (150 µl/well). Endothelin receptor type A (5 µg/ml) expressed and purified in *E. coli* and reconstituted with sarkosyl was immobilized onto the 96-well plate such that its extracellular regions were selectively exposed. AG8 and the four antibodies (MJ-F1, MJ-F2, MJ-F3, and MJ-F4) expressed and purified in animal cells were serially diluted four-fold and allowed to bind to 96-well plates (50 µl/well) at room temperature for 1 h. After each of the 96-well plates was washed 4 times with 0.05% PBST solution (150 µl/well), human IgG(H+L)-HRP conjugate was diluted 1:5,000 with PBS and allowed to bind to the 96-well plate at room temperature for 1 h. After the solution was discarded, the 96-well plate was washed 4 times with 0.05% PBST solution (150 µl/well). The reaction was performed with TMB (Thermo Scientific) (50 µl/well). 20 min later, the reaction was terminated with 4 N H₂SO₄ and the absorbance was measured at 450 nm. Analysis of the ELISA signals revealed that the binding affinities of the control antibody trastuzumab, MJ-F3, and MJ-F4 were too low to be measured whereas the equilibrium dissociation constant of MJ-F2 was 132.8 nM. Particularly, the visible equilibrium dissociation constant of MJ-F1 was 1.41 nM, which was improved by ∼19 times compared to that (26.1) of the antibody AG8 described in the prior art document (Fig. 5, Table 1).

**[Table 1] Calculated equilibrium dissociation constants of the four produced antibodies to human endothelin receptor type A as antigen by ELISA**

| | MJ-F1 | MJ-F2 | AG8 | MJ-F3 | MJ-F4 | Herceptin |
|---|---|---|---|---|---|---|
| K_{D} (nM) | 1.41 | 132.8 | 26.1 | n.d. | n.d. | n.d. |

### <Example 4> Expression and purification of anti-ET_{A} antibodies engineered to have trastuzumab framework regions in animal cells

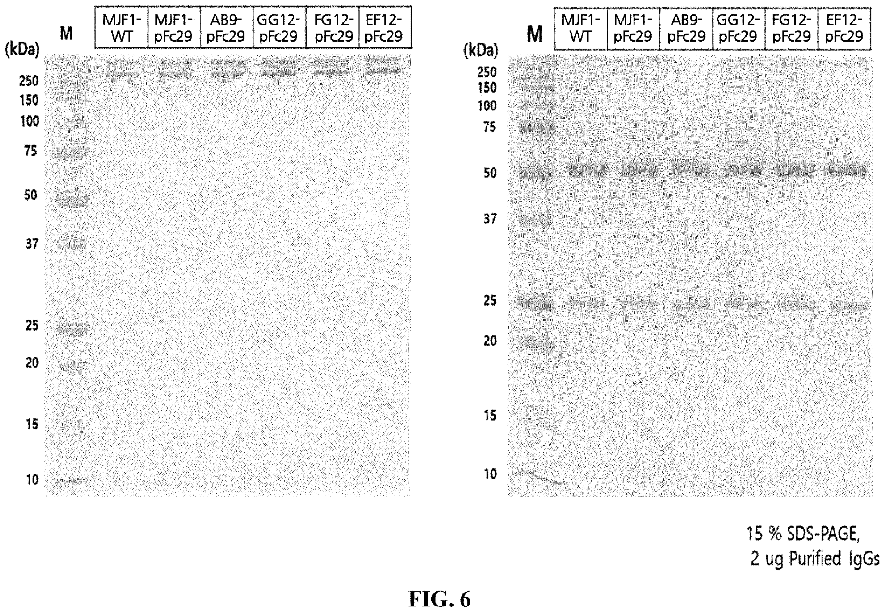

After transformation into *E. coli,* the amplified expression vectors were recovered and transiently transfected into Expi293 cells using a 150 mM NaCl solution containing polyethyleneimine (PEI, Polysciences, USA). Cells were cultured in Freestyle293 expression medium (Life Technologies, USA) at a temperature of 37 °C for 7 days. The expressed cell culture was centrifuged at 6,000 rpm for 20 min. The supernatant was collected and passed through a 0.22 µm filter. The filtrate was allowed to bind to 1 ml of Protein A resin (Amicogen, Korea) at 4 °C for 16 h. The bound resin was washed with 10 CV (column volume) of PBS solution, eluted with 100 mM glycine solution (pH 2.7), and neutralized with 1 M Tris-HCl (pH 8.0). After buffer change with PBS, the sizes and purities of the light and heavy chains of the purified anti-ET_{A} antibodies (MJF1-WT, MJF1-pFc29, FG12-pFc29, and GG12-pFc29) (where the Fc region is wild type or PFc29 as an Fc variant having the sequence set forth in SEQ ID NO: 35) engineered to have trastuzumab framework regions were analyzed by SDS-PAGE under reducing and non-reducing conditions (Fig. 3). The anti-ET_{A} antibodies engineered to have trastuzumab framework regions (MJF1-WT, MJF1-pFc29, FG12-pFc29, and GG12-pFc29) were identified by SDS-PAGE after purification (Fig. 6).

### <Example 5> Analysis of binding affinities between regions of endothelin receptor type A as antigen and the anti-ET_{A} antibodies engineered to have trastuzumab framework regions

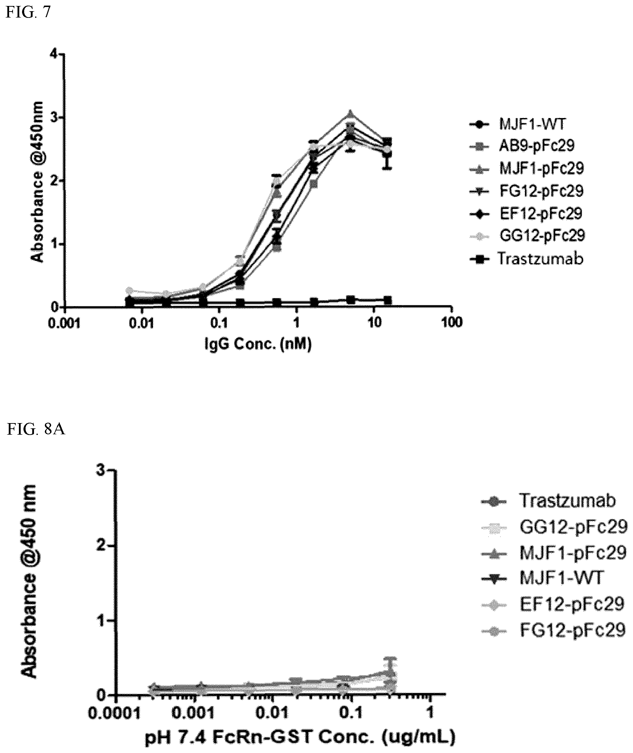

In this example, the binding affinities of the five antibodies (MJF1-WT, MJF1-pFc29, FG12-pFc29, GG12-pFc29, and EF12-pFc29), which were engineered to have the framework regions of trastuzumab selected in Example 1, for endothelin receptor type A were analyzed by ELISA. To this end, human G_{αi3} was diluted with 0.05 M Na₂CO₃ (pH 9.6) as a buffer solution and allowed to bind to 96-well plates (Costar) at a concentration of 5 µg/well at 4 °C for 16 h. Each of the 96-well plates was added to a PBS solution containing 4% skim milk such that the concentration was 5% (w/v), followed by blocking at room temperature for 1 h. After the solution was discarded, the 96-well plate was washed 4 times with a PBST solution (a PBS solution containing 0.5% Tween-20) (150 µl/well). Endothelin receptor type A (5 µg/ml) expressed and purified in *E. coli* and reconstituted with sarkosyl was immobilized onto the 96-well plate such that its extracellular regions were selectively exposed. The five antibodies expressed and purified in animal cells were serially diluted four-fold and allowed to bind to 96-well plates (50 µl/well) at room temperature for 1 h. After each of the 96-well plates was washed 4 times with 0.5% PBST solution (150 µl/well), human IgG(H+L)-HRP conjugate was diluted 1:5,000 with PBS and allowed to bind to the 96-well plate at room temperature for 1 h. After the solution was discarded, the 96-well plate was washed 4 times with 0.5% PBST solution (150 µl/well). The reaction was performed with TMB (Thermo Scientific) (50 µl/well). 20 min later, the reaction was terminated with 4 N H₂SO₄ and the absorbance was measured at 450 nm. Analysis of the ELISA signals revealed that the binding affinity of the control antibody trastuzumab was too low to be measured whereas the equilibrium dissociation constant of MJF1-WT, MJF1-pFc29, AB9-pFc29, FG12-pFc29, EF12-pFc29, and GG12-pFc29 were 0.4873 nM, 0.3637 nM, 0.7706 nM, 0.4809 nM, 0.6044 nM, and 0.2875 nM, respectively (Fig. 7, Table 2).

**[Table 2] Calculated equilibrium dissociation constants of the six antibodies having the framework sequences to endothelin receptor type as antigen A by ELISA**

| | MJF1-WT | MJF1-pFc29 | AB9-pFc29 | FG12-pFc29 | EF12-pFc29 | GG12-pFc29 | Trastuzumab |
|---|---|---|---|---|---|---|---|
| K_{D} (nM) | 0.4873 | 0.3637 | 0.7706 | 0.4809 | 0.6044 | 0.2875 | n.d. |

### <Example 6> Analysis of binding affinities between the anti-ET_{A} antibodies engineered to have trastuzumab framework regions and pH-dependent FcRn

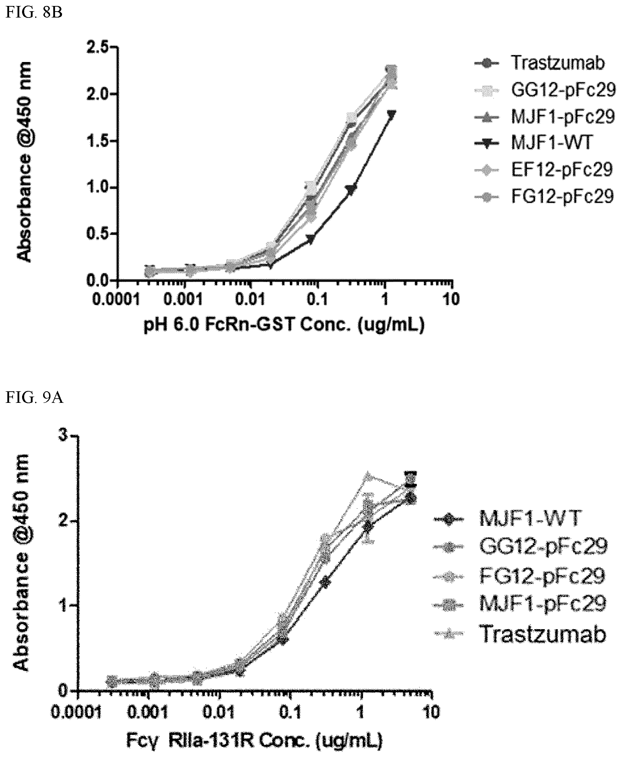

In this example, the binding affinities of the five antibodies (MJF1-WT, MJF1-pFc29, FG12-pFc29, GG12-pFc29, and EF12-pFc29), which were engineered to have the framework regions of trastuzumab selected in Example 1, for human FcRn were analyzed by ELISA. To this end, each of the five antibodies engineered to have trastuzumab framework regions was diluted with 0.05 M Na₂CO₃ (pH 9.6) as a buffer solution and allowed to bind to 96-well plates (Costar) at a concentration of 4 µg/well at 4 °C for 16 h. Each of the 96-well plates was added to a PBS solution containing 4% skim milk such that the concentration was 5% (w/v), followed by blocking at room temperature for 1 h. After the solution was discarded, the 96-well plate was washed 4 times with a PBST solution (a PBS solution containing 0.5% Tween-20) (150 µl/well). A fusion protein of human FcRn and glutathione S-transferase (GST) was serially diluted four-fold and allowed to bind to 96-well plates (50 µl/well) at pH 7.4 and pH 6.0 at room temperature for 1 h. After each of the 96-well plates was washed 4 times with 0.5% PBST solution (150 µl/well), anti-glutathione-HRP conjugate was diluted 1:5,000 with PBST and allowed to bind to the 96-well plate at room temperature for 1 h. After the solution was discarded, the 96-well plate was washed 4 times with 0.5% PBST solution (150 µl/well). The reaction was performed with TMB (Thermo Scientific) (50 µl/well). 20 min later, the reaction was terminated with 4 N H₂SO₄ and the absorbance was measured at 450 nm. The results are shown in Figs. 8A and 8B. Analysis of the ELISA signals revealed that the binding affinities of the five antibodies expressed and purified in animal cells for pH-dependent human FcRn were similar to that of the control antibody trastuzumab, which has a long blood half-life (Figs. 8A and 8B).

### <Example 7> Analysis of binding affinities between the anti-ET_{A} antibodies engineered to have trastuzumab framework regions and human FcyRIIa variants

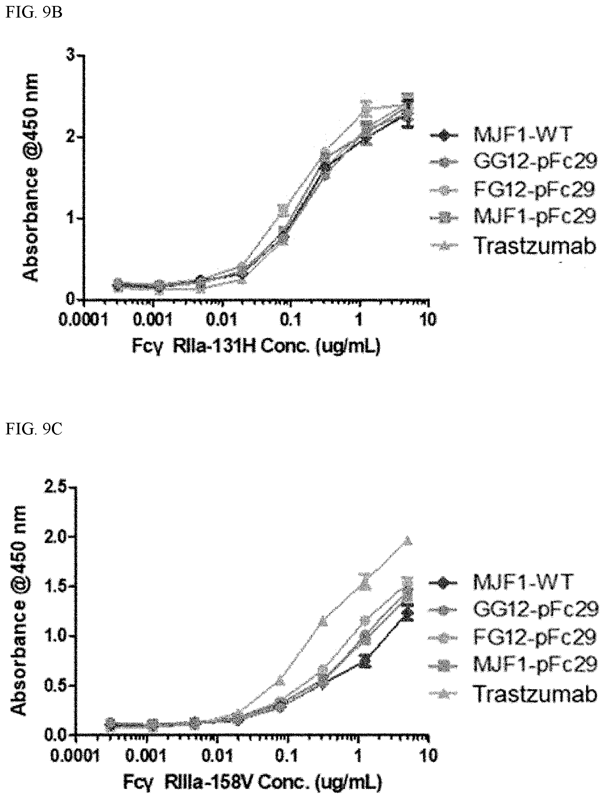

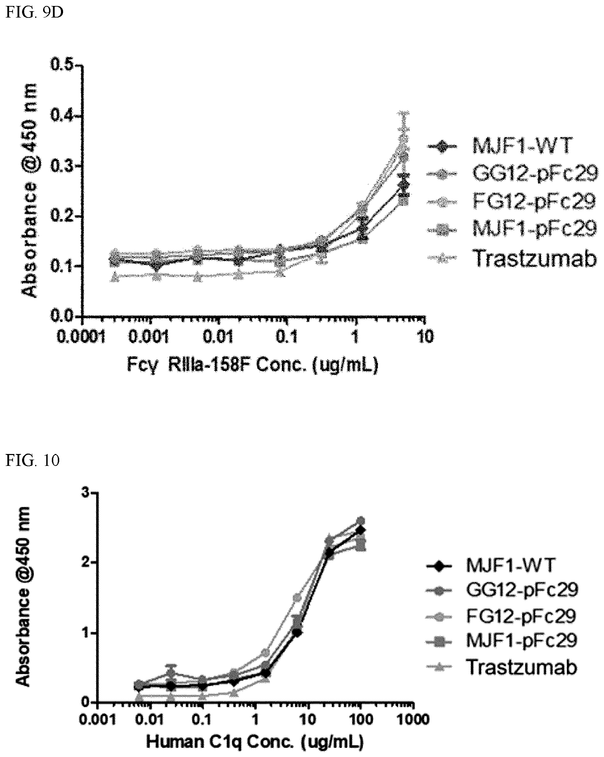

In this example, the binding affinities of the four antibodies (MJF1-WT, MJF1-pFc29, FG12-pFc29, and GG12-pFc29), which were engineered to have the framework regions of trastuzumab selected in Example 1, for human FcyRIIa variants (FcγRIIa-131R, FcγRIIa-131H, FcγRIIa-158V, and FcγRIIa-158F) were analyzed by ELISA. To this end, each of the four antibodies engineered to have trastuzumab framework regions was diluted with 0.05 M Na₂CO₃ (pH 9.6) as a buffer solution and allowed to bind to 96-well plates (Costar) at a concentration of 4 µg/well at 4 °C for 16 h. Each of the 96-well plates was added to a PBS solution containing 4% skim milk such that the concentration was 5% (w/v), followed by blocking at room temperature for 1 h. After the solution was discarded, the 96-well plate was washed 4 times with a PBST solution (a PBS solution containing 0.5% Tween-20) (150 µl/well). GST proteins fused with human FcyRIIa variants were serially diluted four-fold and allowed to bind to 96-well plates (50 µl/well) at room temperature for 1 h. After each of the 96-well plates was washed 4 times with 0.5% PBST solution (150 µl/well), anti-GST-HRP conjugate was diluted 1:5,000 with PBST and allowed to bind to the 96-well plate at room temperature for 1 h. After the solution was discarded, the 96-well plate was washed 4 times with 0.5% PBST solution (150 µl/well). The reaction was performed with TMB (Thermo Scientific) (50 µl/well). 20 min later, the reaction was terminated with 4 N H₂SO₄ and the absorbance was measured at 450 nm. The results are shown in Figs 9A to 9D. Analysis of the ELISA signals revealed that the binding affinities of the four antibodies expressed and purified in animal cells for the human FcyRIIa variants were similar to those of the control antibody trastuzumab (Figs. 9A to 9D).

### <Example 8> Analysis of binding affinities between the anti-ET_{A} antibodies engineered to have trastuzumab framework regions and human C1q

In this example, the binding affinities of the four antibodies (MJF1-WT, MJF1-pFc29, FG12-pFc29, and GG12-pFc29), which were engineered to have the framework regions of trastuzumab selected in Example 1, for human C1q were analyzed by ELISA. To this end, each of the four antibodies engineered to have trastuzumab framework regions was diluted with 0.05 M Na₂CO₃ (pH 9.6) as a buffer solution and allowed to bind to 96-well plates (Costar) at a concentration of 4 µg/well at 4 °C for 16 h. Each of the 96-well plates was added to a PBS solution containing 4% skim milk such that the concentration was 5% (w/v), followed by blocking at room temperature for 1 h. After the solution was discarded, the 96-well plate was washed 4 times with a PBST solution (a PBS solution containing 0.5% Tween-20) (150 µl/well). Human C1q was serially diluted four-fold and allowed to bind to 96-well plates (50 µl/well) at room temperature for 1 h. After each of the 96-well plates was washed 4 times with 0.5% PBST solution (150 µl/well), anti-human C1q-HRP conjugate was diluted 1:400 with PBST and allowed to bind to the 96-well plate at room temperature for 1 h. After the solution was discarded, the 96-well plate was washed 4 times with 0.5% PBST solution (150 µl/well). The reaction was performed with TMB (Thermo Scientific) (50 µl/well). 20 min later, the reaction was terminated with 4 N H₂SO₄ and the absorbance was measured at 450 nm. The results are shown in Fig. 10. Analysis of the ELISA signals revealed that the binding affinities of the four antibodies expressed and purified in animal cells for human C1q were similar to that of the control antibody trastuzumab (Fig. 10).

### <Example 9> Determination of anti-proliferative efficacies of the anti-ET_{A} antibodies engineered to have trastuzumab framework regions against colorectal cancer cells

In this example, a determination was made as to whether the anti-ET_{A} antibodies with engineered framework regions had anti-proliferative efficacies against cells of the colorectal cancer cell lines HT-29 and HCT-116, which are widely used for studies on colorectal cancer cells. The degrees of cell proliferation were confirmed by measuring the amount of DNA in cells using a fluorescent dye (CyQUANT NF, Invitrogen, USA).

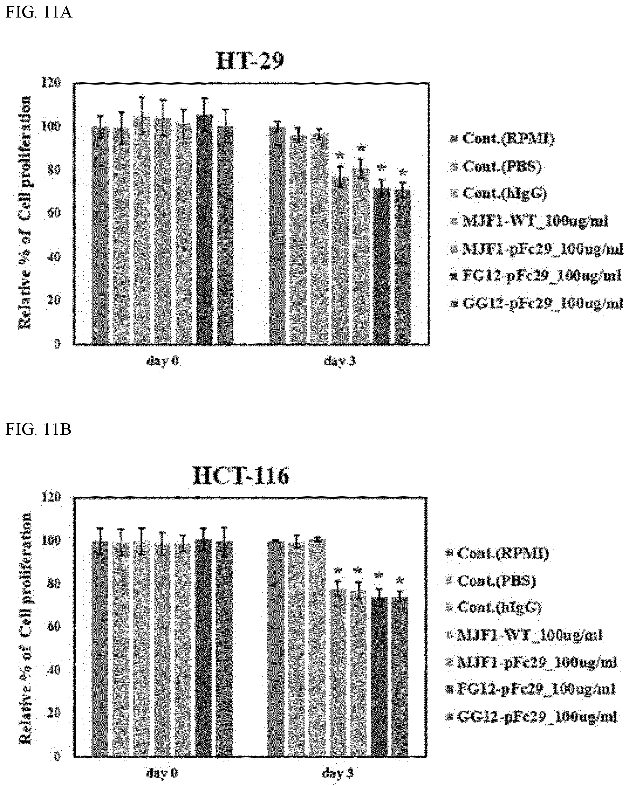

Each of the cell lines HT-29 and HCT-116 was treated with the four anti-ET_{A} antibodies (MJF1-WT, MJF1-pFc29, FG12-pFc29, and GG12-pFc29) engineered to have trastuzumab framework regions and cultured in a cell incubator at 37 °C for 1 h. 72 h after inoculation, cells were treated with a fluorescent dye (CyQUANT NF). The amounts of fluorescence emitted by 485 nm excitation depending on the level of DNA present in cells were measured using a fluorescence spectrophotometer (TECAN, Switzerland) to determine the ability of the anti-ET_{A} antibodies with engineered framework regions to inhibit the proliferation of colorectal cancer cells (Figs. 11A and 11B). As shown in Figs. 11A and 11B, the inventive antibodies effectively inhibited the proliferation of HT-29 and HCT-116 colorectal cancer cell lines.

### <Example 10> Validation of anticancer activities of the anti-ET_{A} antibodies engineered to have trastuzumab framework regions in colorectal cancer animal models

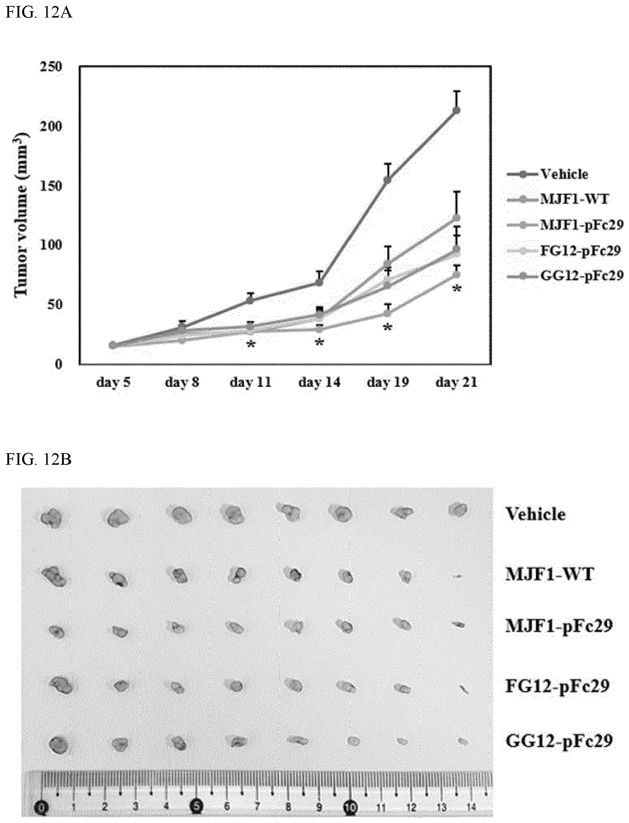

Colorectal cancer cell line HT-29 was injected subcutaneously into the flank region of BALB/c nude mice to establish xenograft models. The cell number was adjusted to 2×10⁶ cells/mouse depending on the amount of PBS (100 µl). From 5 days after injection, each of the inventive antibodies was directly injected into the colorectal cancer tissue at 2-day intervals (200 µg at a time) to validate its anticancer activity. 21 days after injection, the colorectal cancer tissue was excised. The results are shown in Figs. 12A and 12B. In Figs. 12A and 12B, "Vehicle" is a control group injected with PBS alone in the same amount as the antibody.

As shown in Figs. 12A and 12B, the four inventive anti-ET_{A} antibodies (MJF1-WT, MJF1-pFc29, FG12-pFc29, and GG12-pFc29) engineered to have trastuzumab framework regions showed inhibitory effects on the growth of colorectal cancer in the xenograft models of the colorectal cancer cell line HT-29. These results demonstrate that the four inventive anti-ET_{A} antibodies engineered to have trastuzumab framework regions have inhibitory activities on the proliferation of colorectal cancer cells even in animal models, thus being suitable for use in therapeutic agents.

### <Example 11> Determination of anti-proliferative efficacies of the anti-ET_{A} antibodies engineered to have trastuzumab framework regions in pancreatic cancer cells

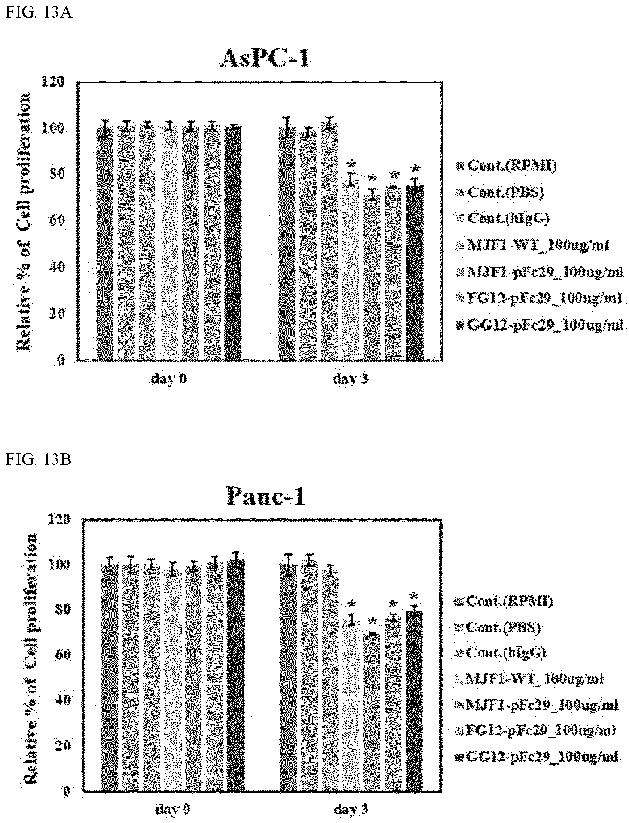

In this example, a determination was made as to whether the anti-ET_{A} antibodies (MJF1-WT, MJF1-pFc29, FG12-pFc29, and GG12-pFc29) with engineered framework regions had anti-proliferative efficacies against cells of the cell lines AsPC-1 and Panc-1, which are widely used for studies on pancreatic cancer cells. To this end, the same procedure as in Example 9 was carried out. The results are shown in Figs. 13A and 13B.

As shown in Figs. 13A and 13B, the anti-ET_{A} antibodies (MJF1-WT, MJF1-pFc29, FG12-pFc29, and GG12-pFc29) with engineered framework regions effectively inhibited the proliferation of cells of the pancreatic cancer cell lines. These results demonstrate that the inventive anti-ET_{A} antibodies (MJF1-WT, MJF1-pFc29, FG12-pFc29, and GG12-pFc29) with engineered framework regions have inhibitory activities on the proliferation of pancreatic cancer cells.

### <Example 12> Determination of anti-proliferative efficacies of the anti-ET_{A} antibodies engineered to have trastuzumab framework regions in gastric cancer cells

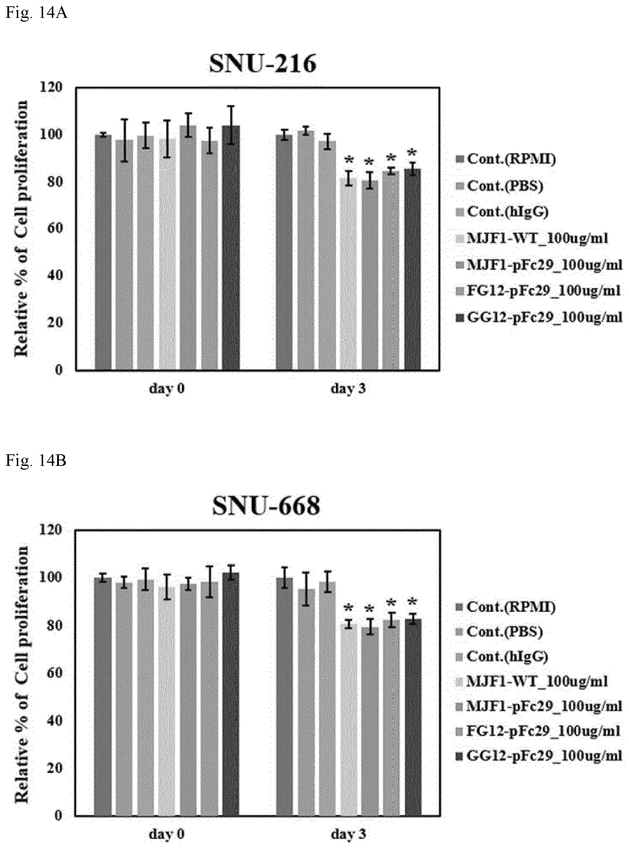

In this example, a determination was made as to whether the anti-ET_{A} antibodies (MJF1-WT, MJF1-pFc29, FG12-pFc29, and GG12-pFc29) with engineered framework regions had anti-proliferative efficacies against cells of the cell lines SNU-216 and SNU-668, which are widely used for studies on gastric cancer cells. To this end, the same procedure as in Example 9 was carried out. The results are shown in Figs. 14A and 14B.

As shown in Figs. 14A and 14B, the anti-ET_{A} antibodies (MJF1-WT, MJF1-pFc29, FG12-pFc29, and GG12-pFc29) with engineered framework regions effectively inhibited the proliferation of cells of the pancreatic cancer cell lines. These results demonstrate that the inventive anti-ET_{A} antibodies (MJF1-WT, MJF1-pFc29, FG12-pFc29, and GG12-pFc29) with engineered framework regions have inhibitory activities on the proliferation of gastric cancer cells.

### <Example 13> Determination of physical properties and characteristics of the anti-ET_{A} antibodies engineered to have trastuzumab framework regions

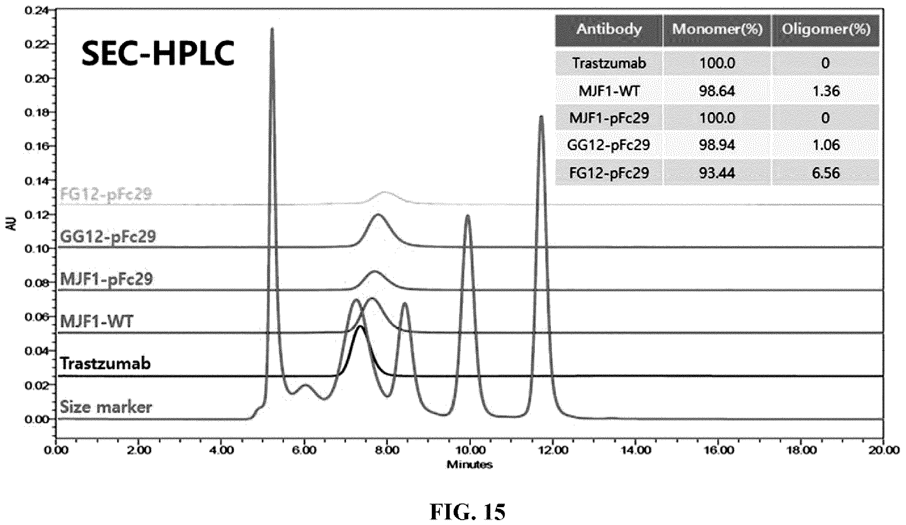

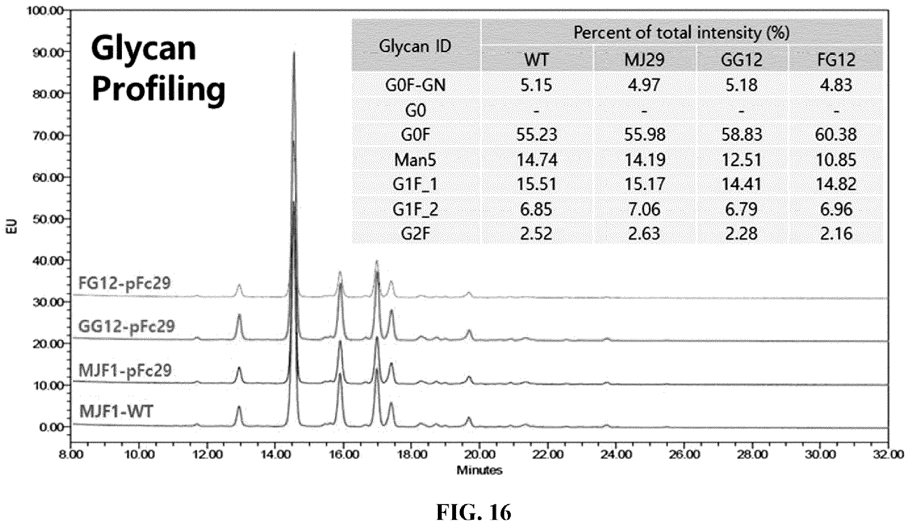

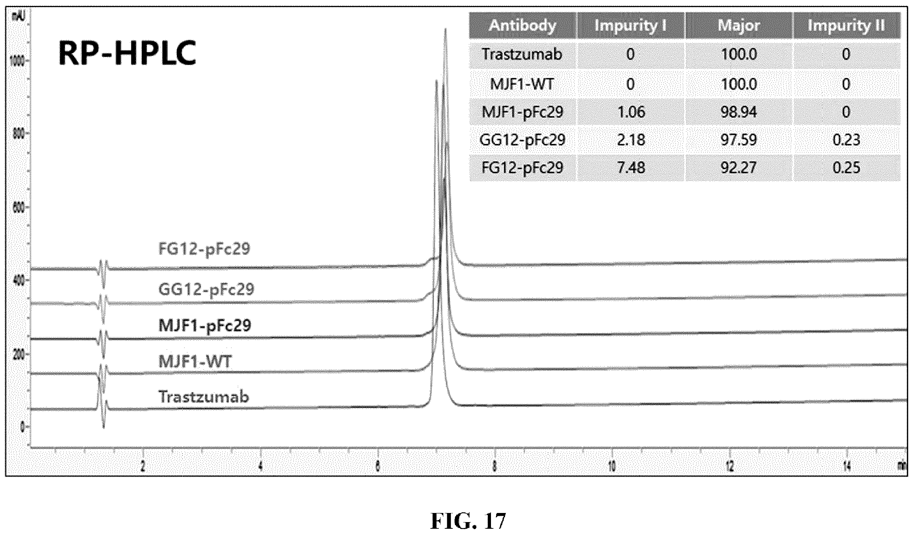

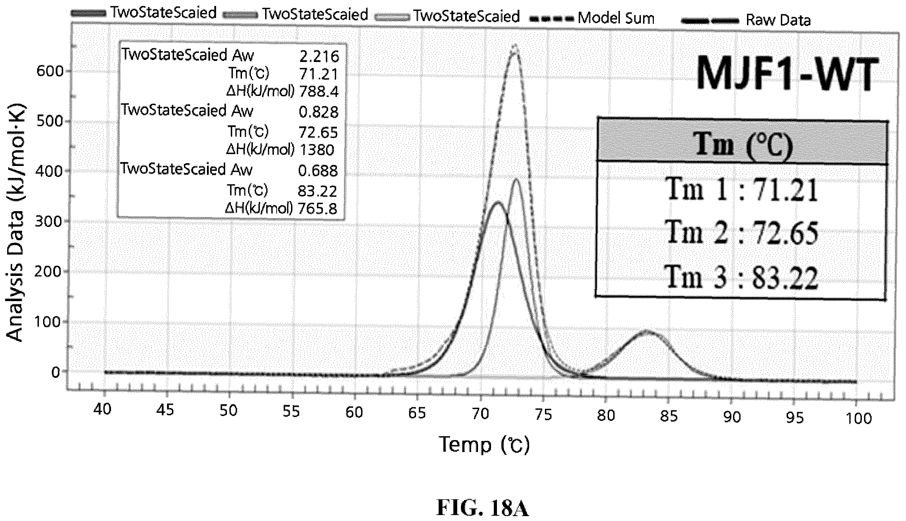

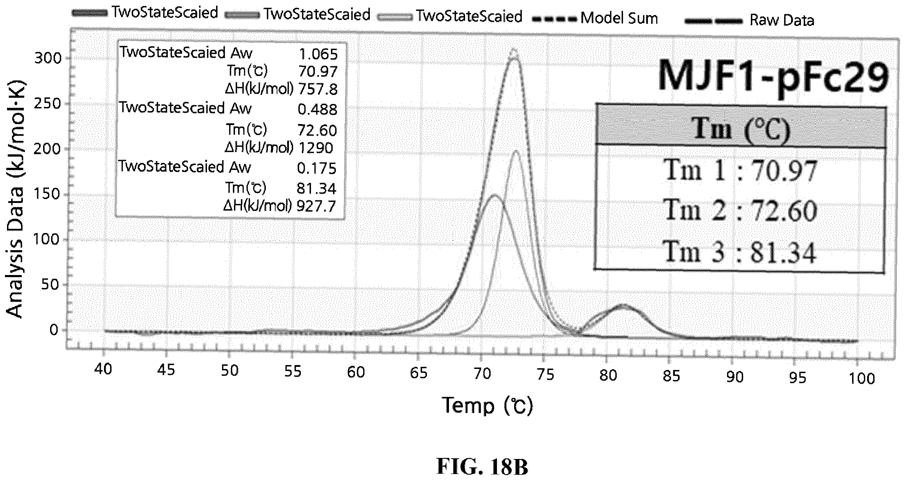

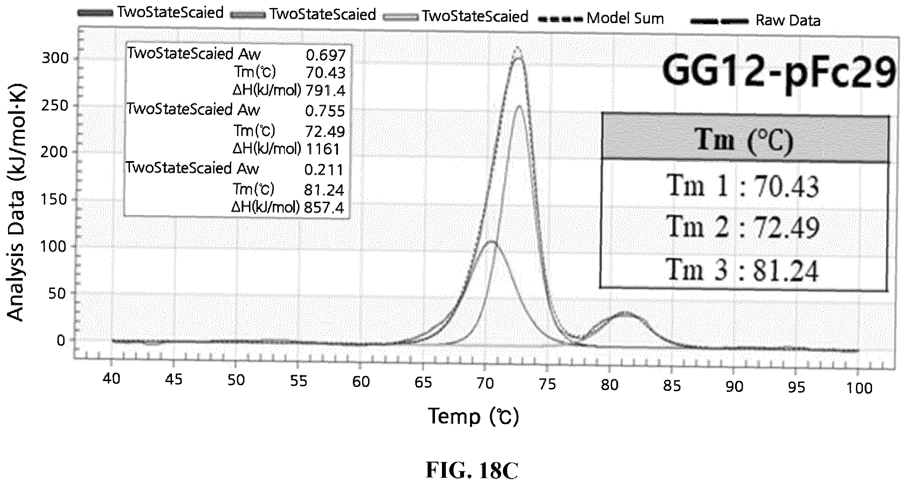

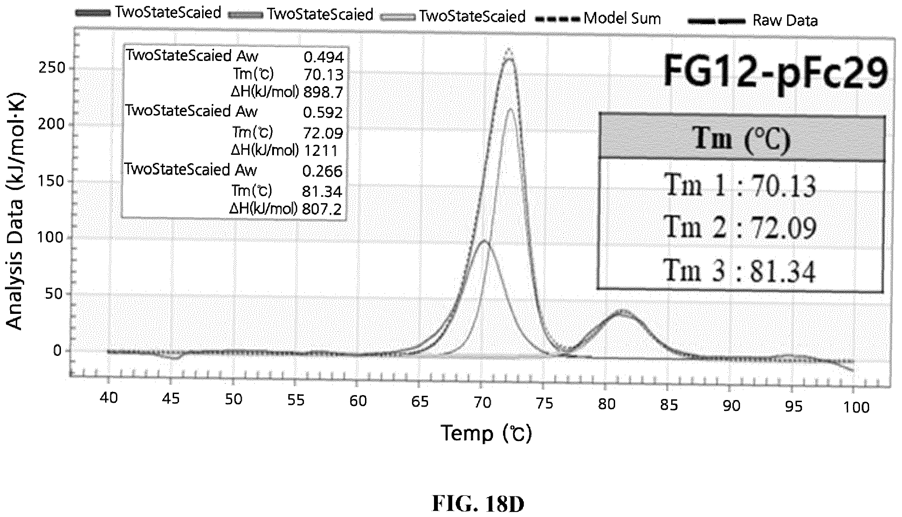

The anti-ET_{A} antibodies (MJF1-WT, MJF1-pFc29, GG12-pFc29, and FG12-pFc29) engineered to have trastuzumab framework regions and the control antibody trastuzumab were separated by SEC-HPLC. The results are shown in Fig. 15. As shown in Fig. 15, the degrees of aggregation were not more than 10%. The glycan profiles of the antibodies were analyzed. The results are shown in Fig. 16. As shown in Fig. 16, the glycan profiles of the developed antibodies did not show significant differences from that of the human IgG standard. The contents of impurities in the antibodies were confirmed by RP-HPLC. The results are shown in Fig. 17. The contents of impurities in all antibodies were found to be < 8%. The thermal stabilities of the discovered antibodies were tested (nanoDSC). As a result, the Tm values of all antibodies were > 70 °C (Figs. 18A to 18D).

Although the particulars of the present invention have been described in detail, it will be obvious to those skilled in the art that such particulars are merely preferred embodiments and are not intended to limit the scope of the present invention. Therefore, the substantial scope of the present invention is defined by the appended claims and their equivalents.

## Claims

1. A monoclonal antibody or an antigen-binding fragment thereof comprising a heavy chain variable region and a light chain variable region and specifically binding to endothelin receptor type A wherein (a) the heavy chain variable region comprises VH-CDR1 comprising the sequence set forth in SEQ ID NO: 25 and VH-CDR2 comprising the sequence set forth in SEQ ID NO: 26, (b) the light chain variable region comprises VL-CDR1 comprising the sequence set forth in SEQ ID NO: 27, VL-CDR2 comprising the sequence set forth in SEQ ID NO: 28, and VL-CDR3 comprising the sequence set forth in SEQ ID NO: 29, and (c) the heavy chain variable region comprises VH-CDR3 comprising the sequence set forth in SEQ ID NO: 30 or VH-CDR3 comprising a sequence comprising mutations at one or more positions selected from the group consisting of amino acid positions 4, 7, 9, and 12 in the sequence of SEQ ID NO: 30.

2. The monoclonal antibody or antigen-binding fragment thereof according to claim 1, wherein the VH-CDR3 of the heavy chain variable region comprises one or more mutations comprising a substitution of the amino acid at position 4 in the sequence of SEQ ID NO: 30 to proline (P), a substitution of the amino acid at position 7 in the sequence of SEQ ID NO: 30 to leucine (L), a substitution of the amino acid at position 9 in the sequence of SEQ ID NO: 30 to valine (V) or a substitution of the amino acid at position 12 in the sequence of SEQ ID NO: 30 to glutamate (E).

3. The monoclonal antibody or antigen-binding fragment thereof according to claim 1, wherein the VH-CDR3 of the heavy chain variable region comprises an amino acid sequence selected from the group consisting of those set forth in SEQ ID NOs: 30, 31, 32, 33, and 34.

4. The monoclonal antibody or antigen-binding fragment thereof according to claim 1, wherein the heavy chain variable region comprises a framework region 1 (VH-FR1) comprising the sequence set forth in SEQ ID NO: 1 or a sequence comprising mutations at one or more positions selected from the group consisting of amino acid positions 16 and 24 in the sequence of SEQ ID NO: 1.

5. The monoclonal antibody or antigen-binding fragment thereof according to claim 4, wherein the VH-FR1 of the heavy chain variable region comprises one or more mutations comprising a substitution of the amino acid at position 16 in the sequence of SEQ ID NO: 1 to arginine (R) or a substitution of the amino acid at position 24 in the sequence of SEQ ID NO: 1 to valine (V).

6. The monoclonal antibody or antigen-binding fragment thereof according to claim 4, wherein the VH-FR1 of the heavy chain variable region comprises an amino acid sequence selected from the group consisting of those set forth in SEQ ID NOs: 1, 2, and 3.

7. The monoclonal antibody or antigen-binding fragment thereof according to claim 1, wherein the heavy chain variable region comprises a framework region 2 (VH-FR2) comprising the sequence set forth in SEQ ID NO: 4 or a sequence comprising mutations at one or more positions selected from the group consisting of amino acid positions 1, 2, 4, 16, and 17 in the sequence of SEQ ID NO: 4.

8. The monoclonal antibody or antigen-binding fragment thereof according to claim 7, wherein the VH-FR2 of the heavy chain variable region comprises one or more mutations selected from the group consisting of mutations comprising a substitution of the amino acid at position 1 in the sequence of SEQ ID NO: 4 to methionine (M), mutations comprising a substitution of the amino acid at position 2 in the sequence of SEQ ID NO: 4 to asparagine (N), mutations comprising a substitution of the amino acid at position 4 in the sequence of SEQ ID NO: 4 to isoleucine (I), mutations comprising a substitution of the amino acid at position 16 in the sequence of SEQ ID NO: 4 to serine (S) or glycine (G), and mutations comprising a substitution of the amino acid at position 17 in the sequence of SEQ ID NO: 4 to alanine (A), tryptophan (W) or serine (S).

9. The monoclonal antibody or antigen-binding fragment thereof according to claim 7, wherein the VH-FR2 of the heavy chain variable region comprises an amino acid sequence selected from the group consisting of those set forth in SEQ ID NOs: 4, 5, 6, and 7.

10. The monoclonal antibody or antigen-binding fragment thereof according to claim 1, wherein the heavy chain variable region comprises a framework region 3 (VH-FR3) comprising the sequence set forth in SEQ ID NO: 8 or a sequence comprising mutations at one or more positions selected from the group consisting of amino acid positions 1, 4, 5, 6, 7, 8, 12, 14, 16, 17, 19, 20, and 21 in the sequence of SEQ ID NO:8.

11. The monoclonal antibody or antigen-binding fragment thereof according to claim 10, wherein the VH-FR3 of the heavy chain variable region comprises one or more mutations selected from the group consisting of mutations comprising a substitution of the amino acid at position 1 in the sequence of SEQ ID NO: 8 to aspartate (D), threonine (T) or asparagine (N), mutations comprising a substitution of the amino acid at position 4 in the sequence of SEQ ID NO: 8 to alanine (A), mutations comprising a substitution of the amino acid at position 5 in the sequence of SEQ ID NO: 8 to aspartate (D), mutations comprising a substitution of the amino acid at position 6 in the sequence of SEQ ID NO: 8 to phenylalanine (F), mutations comprising a substitution of the amino acid at position 7 in the sequence of SEQ ID NO: 8 to glutamate (E), mutations comprising a substitution of the amino acid at position 8 in the sequence of SEQ ID NO: 8 to arginine (R), mutations comprising a substitution of the amino acid at position 12 in the sequence of SEQ ID NO: 8 to phenylalanine (F), mutations comprising a substitution of the amino acid at position 14 in the sequence of SEQ ID NO: 8 to arginine (R) or leucine (L), mutations comprising a substitution of the amino acid at position 16 in the sequence of SEQ ID NO: 8 to asparagine (N) or aspartate (D), mutations comprising a substitution of the amino acid at position 17 in the sequence of SEQ ID NO: 8 to alanine (A), mutations comprising a substitution of the amino acid at position 19 in the sequence of SEQ ID NO: 8 to serine (S), mutations comprising a substitution of the amino acid at position 20 in the sequence of SEQ ID NO: 8 to serine (S), and mutations comprising a substitution of the amino acid at position 21 in the sequence of SEQ ID NO: 8 to leucine (L) or phenylalanine (F).

12. The monoclonal antibody or antigen-binding fragment thereof according to claim 10, wherein the VH-FR3 of the heavy chain variable region comprises an amino acid sequence selected from the group consisting of those set forth in SEQ ID NOs: 8, 9, 10, and 11.

13. The monoclonal antibody or antigen-binding fragment thereof according to claim 1, wherein the heavy chain variable region comprises a framework region 4 (VH-FR4) comprising the sequence set forth in SEQ ID NO: 12.

14. The monoclonal antibody or antigen-binding fragment thereof according to claim 1, wherein the light chain variable region comprises a framework region 1 (VL-FR1) comprising the sequence set forth in SEQ ID NO: 13 or a sequence comprising mutations at one or more positions selected from the group consisting of amino acid positions 4 and 24 in the sequence of SEQ ID NO: 13.

15. The monoclonal antibody or antigen-binding fragment thereof according to claim 14, wherein the VL-FR1 of the light chain variable region comprises one or more mutations comprising a substitution of the amino acid at position 4 in the sequence of SEQ ID NO: 13 to leucine (L) or a substitution of the amino acid at position 24 in the sequence of SEQ ID NO: 13 to serine (S).

16. The monoclonal antibody or antigen-binding fragment thereof according to claim 14, wherein the VL-FR1 of the light chain variable region comprises an amino acid sequence selected from the group consisting of those set forth in SEQ ID NOs: 13, 14, and 15.

17. The monoclonal antibody or antigen-binding fragment thereof according to claim 1, wherein the light chain variable region comprises a framework region 2 (VL-FR2) comprising the sequence set forth in SEQ ID NO: 16 or a sequence comprising mutations at one or more positions selected from the group consisting of amino acid positions 1, 2, and 14 in the sequence of SEQ ID NO: 16.

18. The monoclonal antibody or antigen-binding fragment thereof according to claim 17, wherein the VL-FR2 of the light chain variable region comprises one or more mutations selected from the group consisting of mutations comprising a substitution of the amino acid at position 1 in the sequence of SEQ ID NO: 16 to leucine (L) or methionine (M), mutations comprising a substitution of the amino acid at position 2 in the sequence of SEQ ID NO: 16 to asparagine (N), and mutations comprising a substitution of the amino acid at position 14 in the sequence of SEQ ID NO: 16 to valine (V).

19. The monoclonal antibody or antigen-binding fragment thereof according to claim 17, wherein the VL-FR2 of the light chain variable region comprises an amino acid sequence selected from the group consisting of those set forth in SEQ ID NOs: 16, 17, 18, and 19.

20. The monoclonal antibody or antigen-binding fragment thereof according to claim 1, wherein the light chain variable region comprises a framework region 3 (VL-FR3) comprising the sequence set forth in ID NO: 20 or a sequence comprising mutations at one or more positions selected from the group consisting of amino acid positions 1, 3, 14, and 31 in the sequence of SEQ ID NO: 20.

21. The monoclonal antibody or antigen-binding fragment thereof according to claim 20, wherein the VL-FR3 of the light chain variable region comprises one or more mutations selected from the group consisting of mutations comprising a substitution of the amino acid at position 1 in the sequence of SEQ ID NO: 20 to threonine (T), serine (S) or tyrosine (Y), mutations comprising a substitution of the amino acid at position 3 in the sequence of SEQ ID NO: 20 to glutamine (Q), histidine (H) or glutamate (E), mutations comprising a substitution of the amino acid at position 14 in the sequence of SEQ ID NO: 20 to glycine (G), and mutations comprising a substitution of the amino acid at position 31 in the sequence of SEQ ID NO: 20 to valine (V).

22. The monoclonal antibody or antigen-binding fragment thereof according to claim 20, wherein the VL-FR3 of the light chain variable region comprises an amino acid sequence selected from the group consisting of those set forth in SEQ ID NOs: 20, 21, 22, and 23.

23. The monoclonal antibody or antigen-binding fragment thereof according to claim 1, wherein the light chain variable region comprises a framework region 4 (VL-FR4) comprising the sequence set forth in SEQ ID NO: 24.

24. A nucleic acid molecule encoding the monoclonal antibody or antigen-binding fragment thereof according to claim 1.

25. A vector comprising the nucleic acid molecule according to claim 24.

26. A host cell comprising the vector according to claim 25.

27. A pharmaceutical composition for preventing or treating cancer comprising the monoclonal antibody or antigen-binding fragment thereof according to claim 1, the nucleic acid molecule according to claim 24 or the vector according to claim 25.

28. A pharmaceutical composition for preventing or treating hypertension comprising the monoclonal antibody or antigen-binding fragment thereof according to claim 1, the nucleic acid molecule according to claim 24 or the vector according to claim 25.

29. A method for quantifying endothelin receptor type A in a sample, comprising treating the sample with the monoclonal antibody or antigen-binding fragment thereof according to claim 1.

30. A method for providing information necessary for the diagnosis of a disease caused by overexpression of endothelin receptor type A, comprising (a) separating a sample from a subject, (b) treating the sample with the monoclonal antibody or antigen-binding fragment thereof according to claim 1, and (c) determining whether the expression level of endothelin receptor type A in the sample from the subject is higher than that of endothelin receptor type A in a normal sample.

31. The method according to claim 30, wherein the disease caused by overexpression of endothelin receptor type A is cancer or hypertension.

32. A kit for quantifying endothelin receptor type A comprising the monoclonal antibody or antigen-binding fragment thereof according to claim 1.
